# EUROPÄISCHE PATENTSCHRIFT

(11) **EP 4 239 338 B1**
(45) Veröffentlichungstag und Bekanntmachung des Hinweises auf die Patenterteilung: **18.11.2026**
(21) Anmeldenummer: 22159912.9
(22) Anmeldetag: 03.03.2022
(51) Int. Cl.: G01N 33/86, C12Q 1/56, G01N 33/53

(54) **GLOBALTEST ZUR FESTSTELLUNG DES STATUS DES BLUTGERINNUNGSSYSTEMS**
GLOBAL TEST FOR DETERMINING THE STATUS OF THE BLOOD COAGULATION SYSTEM
ESSAI GÉNÉRAL DESTINÉ À LA DÉTERMINATION DE L'ÉTAT DU SYSTÈME DE COAGULATION SANGUINE

(43) Veröffentlichungstag der Anmeldung: 06.09.2023
(73) Patentinhaber: Siemens Healthcare Diagnostics Products GmbH, 35041 Marburg (DE)
(72) Erfinder: Patzke, Juergen, 35043 Marburg (DE)
(74) Vertreter: Siemens Healthineers Patent Attorneys

(56) Entgegenhaltungen:
- EP-A1- 2 168 985
- US-A- 5 830 681
- CAPECCHI MARCO ET AL: "Relationship between thrombin generation parameters and prothrombin fragment 1 + 2 plasma levels", INTERNATIONAL JOURNAL OF LABORATORY HEMATOLOGY, vol. 43, no. 5, 12 January 2021 (2021-01-12), GB; US, pages 248 - 251, XP55949257, ISSN: 1751-5521, Retrieved from the Internet <URL:https://onlinelibrary.wiley.com/doi/full-xml/10.1111/ijlh.13462> DOI: 10.1111/ijlh.13462
- DUARTE RITA CAROLINA FIGUEIREDO ET AL: "Thrombin generation assays for global evaluation of the hemostatic system: perspectives and limitations", REVISTA BRASILEIRA DE HEMATOLOGIA E HEMOTERAPIA, vol. 39, no. 3, 9 May 2017 (2017-05-09), pages 259 - 265, XP055949444, ISSN: 1516-8484, Retrieved from the Internet <URL:https://www.ncbi.nlm.nih.gov/pmc/articles/PMC5568585/pdf/main.pdf> DOI: 10.1016/j.bjhh.2017.03.009

## Beschreibung

Die vorliegende Erfindung liegt auf dem Gebiet der Blutgerinnungsdiagnostik und betrifft einen neuen globalen Test zur Feststellung des Status des Blutgerinnungssystems eines Individuums anhand der in vitro generierten Menge von F1+2-Peptid.

Die Regulation der Blutgerinnung (Hämostase) erfolgt durch das Zusammenspiel verschiedener Aktivatoren, Inhibitoren sowie positiver und negativer Rückkopplungsmechanismen. Defekte in diesem Gefüge können zu einer Dysbalance im Blutgerinnungssystem führen und entweder eine Hämorrhagie oder eine Thrombose zur Folge haben.

Thrombin, eine Serinprotease, ist das zentrale Enzym der plasmatischen Blutgerinnung, dessen Hauptfunktion die Induktion der Fibrinpolymerisation und damit der Gerinnselbildung ist. Die Bildung von Thrombin wird im Bedarfsfall durch die Aktivierung des enzymatisch inaktiven Vorläufermoleküls Prothrombin in Gang gesetzt. Um im Verletzungsfall den Gerinnungsprozess lokal und zeitlich zu begrenzen, erfolgt eine prompte Drosselung der Gerinnung, u.a. dadurch dass das freie Thrombin von inhibitorischen Faktoren wie z. B. Antithrombin oder α₂-Makroglobulin (α₂M) komplexiert und somit inaktiviert wird. Störungen innerhalb der Prozesse, die die Thrombinbildung oder -inhibition regulieren, können zu hyper- bzw. hypokoagulatorischen Zuständen und damit zu pathologischen Gerinnungsstörungen führen. In der Erfassung der Bildung und der Inhibition des Thrombins liegt also eine immense Aussagekraft über den jeweiligen Gerinnungszustand eines Individuums.

Das einer Probe innewohnende (endogene), im Falle von Plasmaproben plasmaeigene Vermögen (Potenzial) zur Bildung und Inhibition von enzymatisch aktivem, freiem Thrombin wird auch als endogenes Thrombinpotenzial (ETP) bezeichnet. Da alle biologischen Komponenten, die in einem Untersuchungsmaterial enthalten sind und die Bildung und die Inhibition von Thrombin beeinflussen können, das individuelle Thrombinpotenzial einer Probe bedingen, eignet sich die ETP-Bestimmung sowohl als globaler Test, mit dem mehrere Komponenten des Hämostasesystems erfasst werden können, als auch zur Überwachung therapeutischer Maßnahmen. Capecchi, M. et al., Int J Lab Hematol 2021:43:e248-e251 offenbart, dass das endogene Thrombinpotenzial in der Plasmaprobe eines Patienten, also die Thrombingenerierung in vitro nach Zugabe eines Gerinnungsaktivators, mit der Thrombingenerierung in vivo, die durch die Messung der in vivo generierten Menge des F1+2-Peptids in einer Plasmaprobe desselben Patienten bestimmt wird, korreliert.

Ein Parameter, der zur Quantifizierung des endogenen Thrombinpotenzials bevorzugt bestimmt wird, ist das Zeit-/Konzentrationsintegral bzw. die Fläche unter der Thrombinbildungskurve [siehe EP 420332-B1, EP 802986-B1 und Hemker et al. (1993) Thromb. Haemostasis 70: 617-624]. Dieser Parameter ist ein Maß für die Menge und die Aktivität des endogenen Thrombins, welches seit einer Zeit t = 0 in einer Probe von gerinnendem Plasma anwesend war.

Zur Bestimmung des ETP wird in einer Probe gerinnungsfähigen Plasmas die Umsatzkinetik eines Thrombinsubstrats über die Freisetzung eines messbaren Indikators gemessen. Da die Thrombinsubstratkonzentration so eingestellt wird, dass das Substrat im Laufe der Reaktion nicht vollständig aufgebraucht werden kann, verhält sich die Menge an freigesetztem Indikator idealerweise proportional zur enzymatischen Aktivität des im Verlaufe der Gerinnungsreaktion gebildeten Thrombins.

Verschiedene kommerziell erhältliche Testsysteme, die eine automatische Durchführung der ETP-Bestimmung ermöglichen, sind äußerst komplex, weil der komplizierte Prozess der Thrombinaktivierung und -inhibition und die Bestimmung der Thrombinaktivität über die Zeit eine Vielzahl hochspezifischer Anpassungen erfordert, wie z.B. die Auswahl geeigneter Thrombinsubstrate, die Bereitstellung spezifischer Kalibratoren, die Zugabe von Fibrininhibitoren und die Anwendung testspezifischer Algorithmen zur Auswertung der Messwerte (siehe z.B. Kintigh, J. et al. (2018) A review of commercially available thrombin generation assays. Res Pract Thromb Haemost. 2:42-48). Ein weiterer Nachteil der bekannten Testsysteme zur Bestimmung des ETP besteht darin, dass es in der Praxis bislang nicht gelungen ist, die automatisierte ETP-Bestimmung in Vollblut vorzunehmen. Die bekannten kommerziellen Testsysteme eignen sich lediglich für die ETP-Bestimmung in plättchenarmem (PPP) oder plättchenreichem (PRP) Plasma, wobei bei der Verwendung von PRP der Einsatz fluorogener Messmethoden Voraussetzung ist.

Die der Erfindung zugrundeliegende Aufgabe bestand also darin ein Testverfahren bereitzustellen, das hinsichtlich der Aussagekraft zur Funktionalität der Blutgerinnungskaskade bzw. zum Status des Blutgerinnungssystems eines Individuums dem ETP-Testverfahren gleichwertig ist, aber einen vereinfachten Testaufbau, eine vereinfachte Testauswertung und vor allem auch eine Abarbeitung in PRP und Vollblut als Probenmatrix ermöglicht.

Es wurde gefunden, dass die quantitative Bestimmung der F1+2-Menge, die in vitro in einer Probe von Vollblut oder Plasma eines Individuums entsteht, die Feststellung des Status des Blutgerinnungssystems des Individuums gewährleistet und vorteilhafterweise einen vereinfachten Testaufbau, eine vereinfachte Testauswertung und vor allem auch eine Abarbeitung in PRP und Vollblut als Probenmatrix ermöglicht.

Gegenstand der vorliegenden Erfindung ist also ein Verfahren zur Feststellung des Status des Blutgerinnungssystems eines Individuums, wobei das Verfahren -wie die Verfahren zur ETP-Bestimmung aus dem Stand der Technik- folgende Schritte umfasst, die zur Aktivierung des Blutgerinnungssystems in vitro führen:
a. Bereitstellen eines Reaktionsgemisches durch Zugabe eines Gerinnungsaktivators zu einer Probe des Individuums und
b. Inkubation des Reaktionsgemisches.

Das erfindungsgemäße Verfahren zeichnet sich gegenüber dem Stand der Technik dadurch aus, dass das Verfahren ferner die folgenden Schritte umfasst:
c. quantitative Bestimmung der F1+2-Menge in dem Reaktionsgemisch und
d. Feststellung des Status des Blutgerinnungssystems durch Vergleichen der so gemessenen F1+2-Menge in dem Reaktionsgemisch mit einem Referenzwert oder einem Referenzwertbereich, der einen normalen Status des Blutgerinnungssystems eines gesunden Individuums repräsentiert.

Mit dem erfindungsgemäßen Verfahren können verschiedene Zustände des Blutgerinnungssystems erkannt werden.

Ein prokoagulatorischer Status des Blutgerinnungssystems des Individuums, also eine Thromboseneigung, wird dann festgestellt, wenn die in Schritt c) gemessene F1+2-Menge größer ist als der Referenzwert oder der Referenzwertbereich, der einen normalen Status des Blutgerinnungssystems eines gesunden Individuums repräsentiert. Eine solche Thromboseneigung kann zum Beispiel durch zirkulierende präaktivierte Thrombozyten hervorgerufen werden oder durch eine unzureichende therapeutische Antikoagulation verursacht sein.

Ein antikoagulatorischer Status des Blutgerinnungssystems des Individuums, also eine Blutungsneigung, wird dann festgestellt, wenn die in Schritt c) gemessene F1+2-Menge kleiner ist als der Referenzwert oder der Referenzwertbereich, der einen normalen Status des Blutgerinnungssystems eines gesunden Individuums repräsentiert. Eine solche Blutungsneigung kann zum Beispiel durch einen Mangel an Gerinnungsfaktoren oder Thrombozyten hervorgerufen werden oder durch eine zu starke therapeutische Antikoagulation verursacht sein.

Eine Ausnahme hiervon stellt der Lupus anticoagulanz (LA)-Test dar. Bei diesem Test wird bekanntermaßen und paradoxerweise bei limitierenden Phospholipid-Konzentrationen im Testansatz mit einer LA-positiven Probe in vitro eine verringerte Gerinnungsneigung (also eine Blutungsneigung bzw. ein antikoagulatorischer Status) festgestellt, obwohl Lupus anticoagulanz in vivo eine erhöhte Thromboseneigung verursacht. Analog dazu wird auch mit dem erfindungsgemäßen Verfahren in Gegenwart von Lupus antikoagulanz eine verringerte F1+2-Menge im Vergleich zu einem normalen Status des Blutgerinnungssystems eines gesunden Individuums gemessen.

Für die Bereitstellung des Reaktionsgemisches in Schritt a) wird der Probe des Individuums ein Gerinnungsaktivator zur Aktivierung des Gerinnungssystems zugegeben.

Der Begriff "Probe eines Individuums" umfasst insbesondere Körperflüssigkeiten, die einem menschlichen oder tierischen Individuum entnommen sind, vornehmlich plättchenarmes oder plättchenreiches Plasma oder Vollblut.

Unter einem "Gerinnungsaktivator" ist eine Substanz oder ein Substanzgemisch zu verstehen, die/das eine Aktivierung des Blutgerinnungssystems bewirkt. Derartige Substanzen oder Substanzgemische sind dem Fachmann hinlänglich bekannt und umfassen beispielsweise Phospholipide, wie z.B. negativ geladene Phospholipide; Lipoproteine, wie z.B. Thromboplastin; Calciumionen; Proteine, wie z.B. Gewebefaktor, aktivierte Serinproteasen, wie z.B. Faktor IIa (Thrombin), Faktor VIIa, Faktor IXa, Faktor Xa, Faktor XIa oder Faktor XIIa; Schlangengifte, wie z.B. PROTAC^{®} Enzym, Ecarin, Textarin, Noscarin, Batroxobin, Thrombocytin oder Russell's Viper Venom (RVV); Kontaktaktivatoren, wie z.B. Silica, Kaolin, Ellagsäure, Celite, DNA, RNA oder Polyphosphate.

Der Gerinnungsaktivator kann ein Aktivator des plasmatischen Gerinnungssystems sein, vorzugsweise ausgewählt aus der Gruppe Thromboplastin, Faktor IIa, Faktor VIIa, Faktor IXa, Faktor Xa, Faktor XIa, Faktor XIIa, Schlangengifte, negativ geladene Phospholipide, Calciumionen, Gewebefaktor (synonym: Tissue Factor, Faktor III), Silica, Kaolin, Ellagsäure, Celite und Polyphosphate.

Der Gerinnungsaktivator kann ferner ein Plättchenaktivator sein, vorzugsweise ausgewählt aus der Gruppe ADP, Epinephrin, Kollagen, Thrombin-Rezeptor aktivierendes Peptid (TRAP), Thromboxan A2 nachahmendes U46619, Arachidonsäure, Ristocetin, Thrombin, von Willebrand Faktor, Kollagen-bezogenes Peptid (GPVI-Stimulation), Convulxin, Kalzium Ionophor A23187 und Phorbol 12-Myristat 13 Azetat.

Weiterhin kann der Gerinnungsaktivator ein chemischer oder physikalischer Faktor sein, welcher die prokoagulatorischen Eigenschaften von Erythrozyten verstärkt, z.B. deren Freisetzung von ADP und Thromboxan A2, welche wiederum Plättchen aktivieren (Alamin, A.A. (2021) The role of red blood cells in hemostasis. Semin Thromb Hemost 2021; 47: 26-31) oder auch die Präsentation von Phosphatidylserin-Oberflächen bewirken, die prokoagulatorisch wirken (Litvinov, R.I. and Weisel, J.W. (2017) Role of red blood cells in haemostasis and thrombosis. ISBT Sci Ser. 2017 February; 12(1): 176-183).

Weiterhin kann der Gerinnungsaktivator eine Substanz sein, die Leukozyten aktiviert wie zum Beispiel proinflammatorische Cytokine (TNF-α, IL-8), welche bei Neutrophilen einen Zelltod einleiten ("Netosis") und zur Bildung von Neutrophil Extracellular Traps führen, die stark prothrombotisch wirken (Kapoor, S. et al. (2018) The role of neutrophils in thrombosis. Thromb Res. 2018 October; 170: 87-96).

Weiterhin zählen zu den Gerinnungsaktivatoren aktivierende Antikörper im plasmatischen und zellulären System.

Um die durch die Aktivierung der Blutgerinnungskaskade in dem Reaktionsgemisch einsetzende Bildung von Fibrin zu unterbinden, das eine Trübung des Reaktionsgemisches verursacht und dadurch optische Messungen beeinträchtigen könnte, kann dem Reaktionsgemisch in einer bevorzugten Ausführungsform ein Fibrinpolymerisationsinhibitor zugegeben werden. Geeignete Fibrinpolymerisationsinhibitoren sind z.B. Oligopeptide, die die Aneinanderlagerung (Polymerisation) der Fibrinmonomere, die unter der Einwirkung von Thrombin entstehen, inhibieren und damit die Gerinnselbildung verhindern. Besonders geeignete Oligopeptide sind solche der allgemeinen Peptidsequenz GPRP-X-NH₂ , wobei G für die Aminosäure Glycin, P für die Aminosäure L-Prolin, R für die Aminosäure L-Arginin und X für Alanin oder Glycin steht (siehe z.B. EP-B1-456152).

Das durch die Zugabe des Gerinnungsaktivators zur Probe erhaltene Reaktionsgemisch wird inkubiert. Vorzugsweise wird das Reaktionsgemisch in Schritt b) für einen Zeitraum von 5 Sekunden bis 60 Minuten, vorzugsweise von 5 bis 20 Minuten inkubiert. Der Inkubationszeitraum umfasst die Zeitspanne ab Zugabe des Gerinnungsaktivators zur Probe bis zu dem Zeitpunkt, in dem die Gerinnungsreaktion in dem Reaktionsgemisch im Wesentlichen gestoppt wird, zum Beispiel durch die Zugabe eines Verdünnungsmediums, z.B. eines großen Volumens von Pufferlösung oder von Reagenzflüssigkeiten für die Bestimmung von F1+2 (siehe unten), wodurch eine so große Verdünnung des Reaktionsgemisches erzielt wird, das die in dem Reaktionsgemisch stattfindende Gerinnungsreaktion abgestoppt wird. Alternativ kann die Gerinnungsreaktion in dem Reaktionsgemisch durch die Zugabe eines Gerinnungsinhibitors, vorzugsweise eines Gerinnungsinhibitors aus der Gruppe Faktor Xa-Inhibitoren (z.B. Rivaroxaban, Edoxaban, Apixaban, Betrixaban, Heparin-Antithrombin-Komplex), Faktor Va-Inhibitoren (z.B. aktiviertes Protein C) und Calciumchelatoren (z.B. EDTA), gestoppt werden.

Vorzugsweise wird das Reaktionsgemisch nach der Inkubation in Schritt b) durch Zugabe eines Verdünnungsmediums verdünnt und die quantitative Bestimmung der F1+2-Menge in Schritt c) in dem so verdünnten Reaktionsgemisch durchgeführt.

Bevorzugterweise wird das Reaktionsgemisch 1:10 bis 1:400 verdünnt, ganz bevorzugterweise beträgt das Verdünnungsverhältnis 1:40 bis 1:200.

Durch die starke Verdünnung des Reaktionsgemisches bzw. durch die Zugabe eines Gerinnungsinhibitors wird die Entstehung von weiterem F1+2 weitgehend gestoppt. Eine geringfügige Fortsetzung der F1+2-Bildung könnte dann zu Fehlern führen, wenn der Zeitraum ab Verdünnung des Reaktionsgemisches bzw. ab Zugabe eines Gerinnungsinhibitors bis zum Start der F1+2-Bestimmung insgesamt zu lange und zwischen verschiedenen Reaktionsansätzen (Proben, Kontrollen, Kalibratoren) unterschiedlich lang ist. Vorzugsweise ist der Zeitraum zwischen Verdünnung des Reaktionsgemisches bzw. Abstoppen der F1+2-Bildung und der Bestimmung der F1+2-Menge bei jeder Messung gleich groß und beträgt weniger als 2 Minuten (max. 10% relativer Unterschied zwischen einzelnen Bestimmungen).

Ein weiterer Vorteil, der durch die F1+2-Bestimmung in starken Verdünnungen der Reaktionsgemische entsteht, ist das etwaige optische Störfaktoren, wie z.B. Trübungen durch Blutzellen in Vollblut, eliminiert werden.

Vorzugsweise wird die quantitative Bestimmung der F1+2-Menge in dem Reaktionsgemisch oder in dem verdünnten Reaktionsgemisch in Schritt c) mit einem F1+2-spezifischen Immunoassay durchgeführt.

Die quantitative Bestimmung der F1+2-Menge in dem Reaktionsgemisch oder in dem verdünnten Reaktionsgemisch kann auf vielfältige Weise erfolgen. Im Stand der Technik sind eine Reihe von Immunoassays zur quantitativen Bestimmung des Prothrombinfragments F1+2 beschrieben. Das Prothrombinfragment F1+2 ist ein Spaltprodukt des Prothrombins, welches wiederum das Proenzym von Thrombin ist. Das Prothrombin-Protein ist modular aufgebaut und besteht aus einem N-terminalen F1+2-Anteil und einem C-terminalen Thrombin-Anteil. Durch die proteolytische Aktivität des Faktors Xa wird Prothrombin gespalten, so dass je Prothrombin-Molekül (70 kD) ein Thrombin-Molekül (30 kD) unter Freisetzung eines Prothrombin-Fragments F1+2 (35-37 kD) entsteht. Durch die autokatalytische Spaltung von Prothrombin durch Thrombin kann es desweiteren zur Spaltung der Fragmente F1 (23 kDa) und F2 (14 kDa) kommen. Da in vivo Thrombin in freier Form im Blut nicht vorkommt, sondern unmittelbar im Anschluss an seine Bildung an Inhibitoren und an Fibrin gebunden wird, wird das Ausmaß der in vivo-Thrombinbildung indirekt erfasst, z. B. durch die Bestimmung des Aktivierungsmarkers F1+2. Eine Möglichkeit zum Nachweis oder zum Ausschluss einer gesteigerten Thrombinbildung in vivo ist demnach die Bestimmung der F1+2-Konzentration im Plasma. Die im Stand der Technik beschriebenen Immunoassays zur quantitativen Bestimmung von in vivo generiertem Prothrombinfragment F1+2 eignen sich gleichermaßen zur erfindungsgemäßen Bestimmung der infolge der Gerinnungsaktivatorzugabe in dem Reaktionsgemisch, also der in vitro entstandenen F1+2-Menge.

In einer Ausführungsform des erfindungsgemäßen Verfahrens umfasst der F1+2-spezifische Immunoassay die Verwendung eines ersten Antikörpers mit Spezifität für F1+2 und die Verwendung eines zweiten, F1+2-bindenden Antikörpers.

Immunoassays zur quantitativen Bestimmung des Prothrombinfragments F1+2 umfassen typischerweise mindestens einen F1+2-spezifischen Antikörper, der nicht an ungespaltenes Prothrombin bindet. Die Herstellung und Verwendung solcher spezifischer F1+2-Antikörper, die nicht an Prothrombin binden, ist z.B. in EP 303983-A2 (Pelzer et al.), in US 5,830,681 (Hursting et al.) oder in US 2003/0219845 A1 (Ruiz et al.) beschrieben. Für die Spezifität der anti-F1+2-Antikörper ist es wichtig, dass diese an ein Epitop binden, welches zumindest die vier carboxyterminalen Aminosäuren der F2/F1+2-Fragmente (Ile-Glu-Gly-Arg-OH) enthält. Da in der Regel zur Bestimmung der F2/F1+2-Konzentration Sandwich-Immunoassays eingesetzt werden, werden zwei anti-F2/F1+2-Antikörper benötigt. In EP 1541676-A1 (Teigelkamp et al.) sind Antikörper beschrieben, die an ein Epitop auf der N-terminalen Hälfte des F2-Fragments und damit auch an intaktes Prothrombin binden, sich jedoch zur Verwendung als Sekundärantikörper in Kombination mit den F2/F1+2-Neoepitop-spezifischen Primärantikörpern in einem Sandwich-Immunoassay besonders eignen.

Die bekannten Antikörper eignen sich zur Bestimmung der F1+2-Konzentration in heterogenen Immunoassays, bevorzugt in Form eines Sandwich-ELISA-Verfahrens. Dazu werden die F1+2-Neoepitop-spezifischen Antikörper ("Primärantikörper") an eine Festphase gekoppelt und mit der Probe bzw. erfindungsgemäß mit dem Reaktionsgemisch inkubiert, so dass die F1+2-Peptide an die immobilisierten Antikörper binden können. Durch einen Waschschritt werden ungebundene Proteine, insbesondere Prothrombin, entfernt, bevor durch Zugabe einer Antikörperlösung, der zweite, F1+2- und Prothrombin-bindende Antikörper ("Sekundärantikörper") appliziert wird. In der Regel ist dieser Zweitantikörper mit einer signalbildenden Komponente assoziiert, die eine Quantifizierung der F1+2-Menge erlaubt.

In einer anderen Ausführungsform des erfindungsgemäßen Verfahrens umfasst der F1+2-spezifische Immunoassay die Verwendung eines ersten Antikörpers mit Spezifität für F1+2 und die Verwendung eines zweiten Antikörpers mit Spezifität für den Immunkomplex bestehend aus F1+2 und dem ersten Antikörper.

Ein derartiger F1+2-spezifischer Immunoassay ist in EP 2168985-A1 (Althaus et al.) beschrieben. Dabei wird ein Antikörper verwendet, der spezifisch an einen Immunkomplex bindet, welcher Prothrombin-Fragment F1+2, an welches ein Antikörper oder ein Antikörperfragment mit Spezifität für das carboxyterminale Neoepitop des Prothrombin-Fragmentes F1+2 gebunden ist, enthält und wobei der Antikörper aber nicht an das Prothrombin-Fragment F1+2 oder F2 alleine und nicht an den Antikörper oder das Antikörperfragment mit Spezifität für das carboxyterminale Neoepitop des Prothrombin-Fragmentes F2/F1+2 alleine bindet. Dieses Assayformat gewährleistet eine so hohe Spezifität, dass es die Durchführung eines direkten, homogenen Sandwich-Immunoassays ohne Wasch- oder Trennschritte ermöglicht.

In einer bevorzugten Ausführungsform des erfindungsgemäßen Verfahrens umfasst der F1+2-spezifische Immunoassay die Verwendung eines ersten Antikörpers mit Spezifität für F1+2 und die Verwendung eines zweiten, F1+2-bindenden Antikörpers, wobei der erste und der zweite Antikörper jeweils mit einer partikulären Festphase assoziiert sind und die quantitative Bestimmung der F1+2-Menge die folgenden Schritte umfasst:
i. Vermischen des Reaktionsgemisches mit den partikulären Festphasen, die mit dem ersten und zweiten Antikörper assoziiert sind und
ii. Messen der Agglutination der partikulären Festphasen im Reaktionsgemisch.

Unter dem Begriff "partikuläre Festphase" sind im Sinne dieser Erfindung nicht-zelluläre Teilchen zu verstehen, die einen ungefähren Durchmesser von wenigstens 20 nm und nicht mehr als 20 µm aufweisen, üblicherweise zwischen 200 nm und 350 nm, bevorzugt zwischen 250 und 320 nm, besonders bevorzugt zwischen 270 und 290 nm, ganz besonders bevorzugt 280 nm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder einer Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahe kommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-and-shell" Partikel mit einem Kern und einer oder mehreren umhüllenden Schichten. Der Begriff Mikropartikel umfasst beispielsweise Farbstoffkristalle, Metallsolen, Silica-Partikel, Glaspartikel und Magnetpartikel. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z.B. aus Polystyrol, Acrylsäurepolymeren, Methacrylsäurepolymeren, Acrylnitril-Polymeren, AcrylnitrilButadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung der F1+2-bindenden Antikörper an die Latexpartikel erlauben.

Sind in dem Reaktionsgemisch F1+2-Peptide enthalten, binden diese an die F1+2-bindenden Antikörper und bewirken so eine Agglutination der partikulären Festphase.

Die Messung der Agglutination der partikulären Festphase im Reaktionsgemisch kann photometrisch erfolgen, beispielsweise turbidimetrisch oder nephelometrisch. Bindungsteste beruhend auf dem Prinzip der partikelverstärkten Lichtstreuung sind seit etwa 1920 bekannt (zur Übersicht siehe Newman, D.J. et al., Particle enhanced light scattering immunoassay. Ann Clin Biochem 1992; 29: 22-42). Bevorzugterweise werden in diesem Zusammenhang Polystyrolpartikel mit einem Durchmesser von 0,1 bis 0,5 µm, besonders bevorzugt mit einem Durchmesser von 0,15 bis 0,35 µm verwendet. Bevorzugt werden Polystyrolpartikel mit Amin-, Carboxyl- oder Aldehydfunktionen verwendet. Weiterhin bevorzugt werden Schale/Kern-Partikel verwendet. Die Synthese der Partikel und die kovalente Kopplung von Liganden ist z.B. in Peula, J.M. et al., Covalent coupling of antibodies to aldehyde groups on polymer carriers. Journal of Materials Science: Materials in Medicine 1995; 6: 779-785 beschrieben.

Alternativ kann die Messung der Agglutination der partikulären Festphase im Reaktionsgemisch durch die Messung eines Signals erfolgen, das von einem signalbildenden System erzeugt wird, wenn eine erste und eine zweite Komponente des signalbildenden Systems in räumliche Nähe zueinander gebracht werden. In diesem Zusammenhang ist der erste Antikörper mit einer ersten partikulären Festphase assoziiert ist und der zweite Antikörper mit einer zweiten partikulären Festphase assoziiert ist, wobei die erste partikuläre Festphase mit einer ersten Komponente eines signalbildenden Systems assoziiert ist und die zweite partikuläre Festphase mit einer zweiten Komponente des signalbildenden Systems assoziiert ist, und wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden und die Agglutination der partikulären Festphasen in dem Reaktionsgemisch anhand des entstandenen Signals gemessen wird.

In dieser Ausführungsform des erfindungsgemäßen Verfahrens umfasst das signalbildende System mindestens eine erste und eine zweite Komponente, welche so zusammenwirken, dass ein detektierbares Signal entsteht, wenn sie in räumliche Nähe zueinander gebracht werden und dadurch miteinander in Wechselwirkung treten können. Unter einer Wechselwirkung zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z.B. kurzlebigen Singulett-Sauerstoff - zu verstehen. Der Energietransfer kann von einer auf eine andere Komponente erfolgen, möglich ist aber auch eine Kaskade verschiedener Substanzen über die der Energietransfer läuft. Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI^{®} Technologie) oder Photosensitizer und Fluorophor (WO 95/06877) oder radioaktives Iod<125> und Fluorophor (Udenfriend et al. (1985) Proc. Natl. Acad. Sci. 82: 8672 - 8676) oder Fluorophor und Fluoreszenz-Quencher (US 3,996,345). Besonders bevorzugt ist die erste Komponente des signalbildenden Systems ein chemilumineszierendes Agens und die zweite Komponente des signalbildenden Systems ein Photosensitizer oder umgekehrt, und es wird die Chemilumineszenz im Reaktionsgemisch gemessen.

In einer weiteren Ausführungsform, deren Prinzip unter der Abkürzung HISCL bekannt ist, wird zuerst ein erster Antikörper mit Spezifität für F1+2 mit der Probe in Kontakt gebracht, so dass die Bindung an F1+2 schnell erfolgen kann. An den ersten Antikörper ist ein Faktor gebunden (z.B. Biotin), welcher an einen Bindungspartner (z.B. Streptavidin) bindet, der sich auf der Oberfläche von Magnetpartikeln befindet. Diese Magnetpartikel werden in einem zweiten Schritt zugegeben. Nach erfolgter Bindung des Antikörper-F1+2-Komplexes an die Partikel werden die Partikel mittels magnetischer Kräfte an die Oberfläche der Küvette gebunden und durch Waschschritte wird das restliche Reaktionsgemisch entfernt. Daraufhin wird ein zweiter, F1+2- (und ggf. Prothrombin-) bindender Antikörper mit den Magnetpartikeln in Kontakt gebracht. Alternativ kann an dieser Stelle auch der Antikörper aus EP 2168985-A1 (Althaus et al.), der gegen den Immunkomplex aus Anti-F1+2 Antikörper und F1+2 gerichtet ist, genutzt werden. Nach weiteren Waschvorgängen und dem Fachmann bekannten Chemilumineszenz-Verfahren wird die Menge an F1+2 in dem Ansatz bestimmt.

Eine weitere Ausführungsform der HISCL Methode mit ultrahoher Sensitivität ist die Immunkomplex-Transfermethode. Hierbei wird zuerst ein Komplex aus Magnetpartikeln, gebunden an F1+2-Antigen-Antikörper Komplex und einem daran gebundenen Antikörper mit Nachweis-Enyzm (z.B. Alkalische Phosphatase, ALP) gebildet. Nach den Waschvorgängen wird nicht direkt der Chemilumineszenz-Nachweis geführt, sondern die Bindung zwischen Magnetpartikeln und Anti-F1+2-Antikörper wird aufgelöst. Der abgelöste Komplex wird in eine andere Küvette transferiert, während die Magnetpartikel in der ersten Küvette verbleiben. In der zweiten Küvette befinden sich erneut Magnetpartikel, die an den Komplex binden. Nach weiteren Waschvorgängen und dem Fachmann bekannten Chemilumineszenz-Verfahren wird die Menge an F1+2 in dem Ansatz bestimmt.

Das Ergebnis der quantitativen Bestimmung der F1+2-Menge in Schritt c) des erfindungsgemäßen Verfahrens kann z.B. in Form eines Rohwerts des gemessenen Signals der jeweiligen Bestimmungsmethode dargestellt werden (z.B. kilocounts [kcnt]). Alternativ kann das Ergebnis auch als Konzentrationsangabe dargestellt werden; dazu wird der Rohwert des gemessenen Signals unter Benutzung eines F1+2-Kalibrators mit bekannter F1+2 Konzentration in eine F1+2-Konzentration (z.B. pmol/L) umgewandelt. Unter Verwendung von Kalibratoren mit normalen oder prokoagulatorischen oder antikoagulatorischen Eigenschaften kann entweder der Rohwert des gemessenen Signals oder die abgeleitete Konzentration in % der Norm umgerechnet werden.

Der Status des Blutgerinnungssystems wird schließlich erfindungsgemäß festgestellt durch Vergleichen der so gemessenen F1+2-Menge in dem Reaktionsgemisch mit einem Referenzwert oder einem Referenzwertbereich, der einen normalen Status des Blutgerinnungssystems eines gesunden Individuums repräsentiert (Schritt d)).

Als Referenzwert eignet sich die F1+2-Menge, die mit demselben Verfahren in Reaktionsgemischen gemessen wird (oder vorab gemessen wurde), die Proben von Individuen oder von Proben von Probenpools von mehreren Individuen enthalten, die bekanntermaßen einen normalen Status des Blutgerinnungssystems aufweisen. Üblicherweise wird zur Bestimmung eines Referenzwertes oder eines Referenzwertbereiches in einer Vielzahl von Proben von gesunden Spendern, die bekanntermaßen einen normalen Status des Blutgerinnungssystems aufweisen, die F1+2-Menge im Reaktionsgemisch gemessen und dann mit der F1+2-Menge einer Vielzahl von Proben von Spendern mit bekanntermaßen prokoagulatorischem Status des Blutgerinnungssystems und/oder von Spendern mit bekanntermaßen antikoagulatorischem Status des Blutgerinnungssystems verglichen. Ein Referenzwert kann beispielsweise dann ein Grenzwert sein, der die Differenzierung von Individuen mit normalem Status des Blutgerinnungssystems von Individuen mit prokoagulatorischem Status des Blutgerinnungssystems beziehungsweise von Individuen mit normalem Status des Blutgerinnungssystems von Individuen mit antikoagulatorischem Status des Blutgerinnungssystems ermöglicht. Überschreitet die in einem Reaktionsgemisch gemessene F1+2-Menge den vorbestimmten Referenzwert, erlaubt dies das Feststellen eines prokoagulatorischen Status des Blutgerinnungssystems des Individuums; unterschreitet die in einem Reaktionsgemisch gemessene F1+2-Menge den vorbestimmten Referenzwert, erlaubt dies das Feststellen eines antikoagulatorischen Status des Blutgerinnungssystems des Individuums. Die beschriebenen Referenzwerte können der obere und der untere Grenzwert eines Referenzwertbereiches sein, der einen Bereich für die in einem Reaktionsgemisch gemessene F1+2-Menge angibt, die einem normalen Status des Blutgerinnungssystems eines Individuums entspricht.

In einer besonderen Ausführungsform des erfindungsgemäßen Verfahrens wird in der Probe zusätzlich in einem zweiten Testansatz die Menge an F1+2 in der Probe bestimmt, ohne dass die Probe mit einem Gerinnungsaktivator in Kontakt gebracht wird, also ohne dass die Thrombin- und damit F1+2-Bildung in der Probe induziert wird. Dazu wird die Probe mit einem Puffer anstelle des Gerinnungsaktivators vermischt und danach gleichermaßen inkubiert und verdünnt wie im erfindungsgemäßen Testansatz. Die so bestimmte F1+2-Menge entspricht der F1+2-Menge, die bereits ohne Gerinnungsaktivierung in der Probe vorhanden ist. Durch Differenzbildung der Testergebnisse aus dem ersten Testansatz (mit Gerinnungsaktivator) und dem Testergebnis aus dem zweiten Testansatz (ohne Gerinnungsaktivator) wird vermieden, dass etwaige hohe F1+2-Mengen, die bereits ohne die Zugabe eines Gerinnungsaktivators in der Probe vorliegen, eine erhöhte in vitro F1+2-Generierungskapazität vortäuschen.

Offenbart, aber nicht beansprucht ist ein Testkit zur Feststellung des Status des Blutgerinnungssystems eines Individuums, wobei das Testkit mindestens folgende Komponenten enthält:
A. ein Reagenz enthaltend einen Gerinnungsaktivator und
B. ein oder mehrere Reagenzien zur quantitativen Bestimmung von F1+2.

Das Gerinnungsaktivator-enthaltende Reagenz enthält eine der weiter oben beschriebenen Substanzen oder ein Substanzgemisch, die eine Aktivierung des Blutgerinnungssystems bewirken. Das Gerinnungsaktivator-enthaltende Reagenz ist zur Bereitstellung des Reaktionsgemisches mit der Körperflüssigkeitsprobe des Individuums vorgesehen.

Die ein oder mehreren Reagenzien zur quantitativen Bestimmung von F1+2 enthalten F1+2-bindende Antikörper (wie weiter oben beschrieben) und Komponenten eines signalbildenden Systems (ebenfalls weiter oben beschrieben). Die ein oder mehreren Reagenzien zur quantitativen Bestimmung von F1+2 sind zur quantitativen Bestimmung der in vitro-generierten F1+2 Menge in dem Reaktionsgemisch aus Körperflüssigkeitsprobe des Individuums und Gerinnungsaktivator-enthaltendem Reagenz vorgesehen.

Die Reagenzien des Testkits können in flüssiger oder lyophilisierter Form bereitgestellt werden. Für den Fall, dass einige oder alle Reagenzien des Testkits als Lyophilisate vorliegen, kann das Testkit zusätzlich die zur Lösung der Lyophilisate erforderlichen Lösemittel enthalten, wie z.B. destilliertes Wasser oder geeignete Puffer.

Als Kontrollen oder Kalibratoren für das erfindungsgemäße Verfahren eignen sich Plasma- bzw. Vollblutproben mit definierter Prothrombinkonzentration. Die Herstellung derartiger Kontroll- bzw. Kalibratormaterialien ist z.B. in EP 1544621-A1 beschrieben.

In einer Ausführungsform enthält ein Testkit zusätzlich
C. ein erstes Kalibratormaterial, das einen normalen Status des Blutgerinnungssystems eines gesunden Individuums repräsentiert, und optional
D. mindestens ein zweites Kalibratormaterial, das einen prokoagulatorischen oder einen antikoagulatorischen Status des Blutgerinnungssystems eines Individuums repräsentiert.

Das Kalibratormaterial, das einen normalen Status des Blutgerinnungssystems eines gesunden Individuums repräsentiert, kann z.B. aus einem Plasmapool mehrerer gesunder Spender bestehen ("Normalplasma").

Das Kalibratormaterial, das einen prokoagulatorischen Status des Blutgerinnungssystems eines Individuums repräsentiert, kann z.B. aus einem Plasmapool mehrerer gesunder Spender oder mehrerer Spender mit der Prothrombin Mutation G20210A bestehen, dem definierte Mengen eines oder mehrerer Gerinnungsfaktoren (z.B. Prothrombin, Faktor V, Faktor X) zugegeben wurden.

Das Kalibratormaterial, das einen antikoagulatorischen Status des Blutgerinnungssystems eines Individuums repräsentiert, kann z.B. aus einem mit Puffer verdünnten Plasmapool mehrerer gesunder Spender oder aus einem Prothrombinmangelplasma oder aus einer Mischung verschiedener Mangelplasmen bestehen.

### FIGURENBESCHREIBUNG

- FIG. 1: zeigt ein Diagramm, in dem der lineare Zusammenhang zwischen gemessener F1+2-Menge [kcnt] und Verdünnung (unverdünnt, 1:2 und 1:4) der verschiedenen Probentypen (Vollblut, plättchenreiches Plasma (PRP), plättchenarmes Plasma (PPP) deutlich wird.
- FIG. 2: zeigt ein Diagramm, in dem die Kalibrationskurve für den erfindungsgemäßen Test zur Bestimmung der in vitro-generierten F1+2-Menge gezeigt ist.
- FIG. 3: zeigt ein Diagramm, in dem die erfindungsgemäß bestimmte F1+2-Generierung (% der Norm) mit dem ETP (Parameter = Cmax; % der Norm) in verschiedenen Proben verglichen wird.
- FIG. 4: zeigt ein Diagramm, in dem die erfindungsgemäß bestimmte F1+2-Generierung (% der Norm) mit dem ETP (Parameter = AUC; % der Norm) in verschiedenen Proben verglichen wird.
- FIG. 5: zeigt vier Diagramme, in denen das erfindungsgemäß bestimmte F1+2 Generierungspotenzial und das endogene Thrombinpotenzial (ETP) von Plasmaproben mit unterschiedlichen Gerinnungsfaktormangelzuständen (Faktor II, VII, IX und X) vergleichend dargestellt ist.
- FIG. 6: zeigt ein Diagramm, in dem das erfindungsgemäß bestimmte F1+2 Generierungspotenzial mit Variationen der Inkubationszeiten und die mit einem Anti-FXa-Test bestimmte Rivaroxaban-Menge in Plasmaproben mit unterschiedlicher Rivaroxaban-Konzentration vergleichend dargestellt ist.
- FIG. 7: zeigt ein Diagramm, in dem das erfindungsgemäß bestimmte F1+2 Generierungspotenzial (und die mit einem Anti-IIa-Test bestimmte Dabigatran-Menge in Plasmaproben mit unterschiedlicher Dabigatran-Konzentration vergleichend dargestellt ist.
- FIG. 8: zeigt ein Diagramm, in dem das erfindungsgemäß bestimmte F1+2 Generierungspotenzial in einer Plasmaprobe mit 120 ng/mL Dabigatran bei unterschiedlichen Inkubationszeiten dargestellt ist.
- FIG. 9: zeigt zwei Diagramme, in denen die erfindungsgemäß bestimmte F1+2-Generierung (% der Norm) unter Anwendung einer nur 5minütigen Inkubationszeit nach Zugabe aller Gerinnungsaktivatoren mit dem ETP (Parameter = Cmax; % der Norm, linkes Diagramm; Parameter = AUC; % der Norm, rechtes Diagramm) in verschiedenen Proben verglichen wird.
- FIG. 10: zeigt Diagramme, in denen die erfindungsgemäß bestimmte F1+2-Generierung (kcnt) in mit Kollagen präaktivierten Vollblut- (WB), PRP- und PPP-Proben dreier Blutspender gezeigt ist.
- FIG. 11: zeigt ein Diagramm, in dem die erfindungsgemäß bestimmte F1+2-Generierung (kcnt) in mit Kollagen präaktivierten Plasmaproben mit unterschiedlicher Thrombozytenzahl gezeigt ist.
- FIG. 12: zeigt ein Diagramm, in dem ein Lupus antikoagulanz-Gerinnungstest mit dem erfindungsgemäßen Lupus antikoagulanz-F1+2 Generierungstest verglichen wird. Im Ansatz "Original" ist die Probe unverändert eingesetzt, im Ansatz "Mischung" ist die Probe 1:1 mit einem Normalplasma gemischt.
- FIG. 13: zeigt ein Diagramm, in dem die erfindungsgemäß bestimmte F1+2-Generierung (kcnt) in Vollblut (WB), PRP und PPP mit/ohne Hemmung der Thrombozyten durch Prostaglandin E1 (PGE1) und mit/ohne nachfolgende Aktivierung der Thrombozyten durch Kollagen gezeigt ist.

### BEISPIELE

### BEISPIEL 1: Bestimmung der Menge von in vitro-generiertem F1+2-Peptid in Plasma- und Vollblutproben

Proben von Vollblut oder Plasma (PRP oder PPP) wurden mit den Komponenten des INNOVANCE ETP Assays (Siemens Healthcare Diagnostics Products GmbH, Deutschland) wie folgt gemischt:
87 µL ETP Puffer
+ 40 µL ETP Reagenz
265 Sekunden Inkubation
+ 108 µL Probe
440 Sekunden Inkubation
+ 15 µL Innovin Reagenz (lipidierter Tissue Factor; Siemens Healthcare Diagnostics Products GmbH, Deutschland)
+ 10 µL ETP CaCl₂-Lösung
20 Minuten Inkubation bei 37°C
1:100-Verdünnung des Reaktionsgemisches durch Zugabe von Owrens Veronal Puffer (OVB-Puffer).

5 µL des verdünnten Reaktionsgemisches wurden mit
- mit einem ersten Reagenz ("Reagenz BA") enthaltend einen ersten biotinylierten monoklonalen Antikörper mit Spezifität für das Neoepitop von F1+2; und anschließend
- einem zweiten Reagenz ("Reagenz Chemibeads") enthaltend einen zweiten monoklonalen Antikörper mit Spezifität für den Immunkomplex bestehend aus dem ersten Antikörper und daran gebundenem F1+2-Peptid, welcher an Latexpartikel gekoppelt ist, die mit einer Chemilumineszenzverbindung assoziiert sind (Chemibeads); und
- mit einem dritten Reagenz ("Reagenz Sensibeads") enthaltend Streptavidin beschichtete Latexpartikel, die mit einem Photosensibilisator assoziiert sind (Sensibeads),
vermischt.

Nach einer weiteren Inkubation des Reaktionsansatzes für einen Zeitraum von 6 Minuten wurde das Chemilumineszenzsignal in der willkürlichen Einheit kilocounts (kcnt) gemessen.

Die hier verwendete LOCI^{®} Technologie beruht darauf, dass eine auf Latexpartikeln gekoppelte Chemilumineszenzverbindung (Chemibeads, CB) und ein auf Latexpartikeln gekoppelter Photosensibilisator (Sensibeads, SB) durch Bindung an einen Analyten (hier: F1+2) in eine räumliche Nähe zueinander gebracht werden, so dass Singulett-Sauerstoff, der vom Photosensibilisator erzeugt wird, die Chemilumineszenzverbindung anregen kann. Die Menge der erzeugten Chemilumineszenz korreliert mit der Analytmenge.

Auf diese Weise wurden Proben von drei offensichtlich gesunden, normalen Spendern untersucht. Dabei wurden für jede Probe Doppelbestimmungen durchgeführt und alle Ergebnisse gemittelt (Tab. 1). Die Ergebnisse zeigen, dass in Gegenwart von Tissue Factor, Lipiden und CaCl₂ aus den INNOVANCE ETP Reagenzien und ohne Zugabe einen Plättchenaktivators das F1+2-Generierungspotenzial bei PRP und PPP nahezu gleich hoch ist. Da bedingt durch den Hämatokrit von 36 bis 53% (Normalbereich Frauen und Männer) das tatsächlich zur Messung von F1+2 zur Verfügung stehende Plasmavolumen in Vollblut nur 47 bis 64% beträgt, ist der ermittelte Wert von 1242 kcnt in Vollblut im Vergleich zu 2111 kcnt in Plasma sehr plausibel. Dies belegt gleichzeitig, dass durch die starke Verdünnung gewährleistet ist, dass die durch die hohe Thrombozytenzahl in PRP und durch die hohe Gesamtzellzahl in Vollblut hervorgerufene Trübung die F1+2 Bestimmung nicht erheblich stört.

**Tabelle 1**

| **Probe** | **F1+2-Menge [kcnt]** |
|---|---|
| **Vollblut** | 1242 |
| **PRP** | 2009 |
| **PPP** | 2111 |

Zwecks Untersuchung der Abhängigkeit der in vitro-generierten F1+2-Menge von den zur Verfügung stehenden koagulatorischen Faktoren wurden die Proben 1:2 und 1:4 mit OVB-Puffer vorverdünnt (Tab. 2).

**Tabelle 2**

| **Spender** | **Probe** | **Verdünnung** | **F1+2-Menge [kcnt]** | | **Mittelwert [kcnt]** | **SD [kcnt]** | **VK (%)** |
|---|---|---|---|---|---|---|---|
| | | | **1** | **2** | | | |
| **1** | **Vollblut** | 1:1 | 1367 | 1517 | 1442 | 106,0 | 7,3 |
| | | 1:2 | 622 | 615 | 618 | 4, 9 | 0, 8 |
| | | 1:4 | 298 | 291 | 294 | 5,1 | 1,7 |
| **2** | | 1:1 | 1213 | 1278 | 1246 | 45,8 | 3,7 |
| | | 1:2 | 538 | 473 | 505 | 45,8 | 9,1 |
| | | 1:4 | 231 | 217 | 224 | 10,1 | 4,5 |
| **3** | | 1:1 | 1041 | 1033 | 1037 | 5, 8 | 0, 6 |
| | | 1:2 | 481 | 485 | 483 | 3, 2 | 0,7 |
| | | 1:4 | 218 | 215 | 217 | 2,0 | 0, 9 |
| **1** | **PRP** | 1:1 | 2379 | 2498 | 2439 | 84,2 | 3,5 |
| | | 1:2 | 1172 | 1083 | 1127 | 62, 9 | 5, 6 |
| | | 1:4 | 521 | 561 | 541 | 28,4 | 5,2 |
| **2** | | 1:1 | 1984 | 1901 | 1942 | 58,7 | 3,0 |
| | | 1:2 | 957 | 895 | 926 | 44,2 | 4,8 |
| | | 1:4 | 451 | 407 | 429 | 31,0 | 7,2 |
| **3** | | 1:1 | 1689 | 1603 | 1646 | 60,8 | 3,7 |
| | | 1:2 | 728 | 774 | 751 | 32,0 | 4,3 |
| | | 1:4 | 372 | 356 | 364 | 11,2 | 3,1 |
| **1** | **PPP** | 1:1 | 2705 | 2478 | 2592 | 160,9 | 6,2 |
| | | 1:2 | 1404 | 1215 | 1309 | 133,3 | 10,2 |
| | | 1:4 | 592 | 574 | 583 | 13,2 | 2,3 |
| **2** | | 1:1 | 2044 | 1888 | 1966 | 110,5 | 5,6 |
| | | 1:2 | 951 | 942 | 947 | 5,9 | 0,6 |
| | | 1:4 | 451 | 423 | 437 | 19,7 | 4,5 |
| **3** | | 1:1 | 1865 | 1687 | 1776 | 125,8 | 7,1 |
| | | 1:2 | 884 | 822 | 853 | 44,3 | 5,2 |
| | | 1:4 | 404 | 379 | 391 | 17,3 | 4,4 |

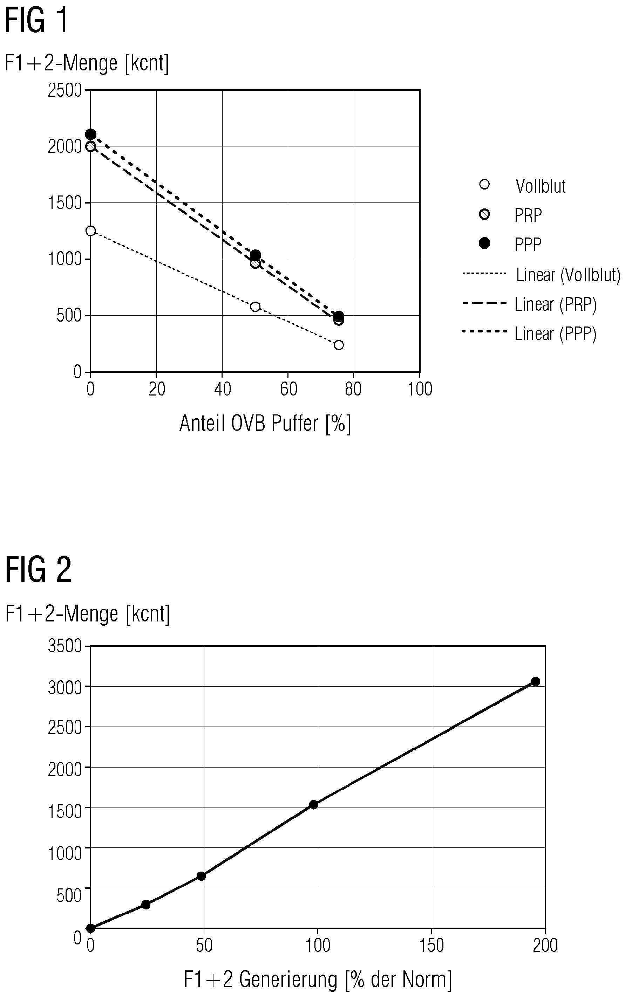

Es wurde ein linearer Zusammenhang zwischen dem Anteil an Probe (folglich an Gerinnungsfaktoren) und der in vitro gebildeten F1+2-Menge in allen drei Probentypen festgestellt. Dies belegt die Verdünnungslinearität der Testergebnisse und bei Vollblut und PRP als Probe damit auch die Abwesenheit von Störungen, die allein durch die Anwesenheit von Zellen (z.B. der sogenannte "inner filter effect") und die Trübung der Probe hervorgerufen werden könnten (FIG. 1).

### BEISPIEL 2: Vergleich des erfindungsgemäßen F1+2 Generierungs-Assays mit einem Thrombingenerierungs-Assay (Endogenes Thrombinpotenzial)

Zur Durchführung des erfindungsgemäßen Verfahrens zur Bestimmung der in vitro-generierten F1+2-Menge wurden Plasmaproben mit den Komponenten des INNOVANCE ETP Assays (Siemens Healthcare Diagnostics Products GmbH, Deutschland) wie folgt gemischt:
108 µL Probe
+ 82 µL ETP Puffer
+ 35 µL ETP Reagenz
150 Sekunden Inkubation bei 37°C
+ 15 µL Innovin Reagenz (lipidierter Tissue Factor)
+ 10 µL ETP CaCl₂-Lösung
20 Minuten Inkubation bei 37°C
1:100-Verdünnung des Reaktionsgemisches durch Zugabe von OVB-Puffer.

5 µL des verdünnten Reaktionsgemischs wurden wie in Beispiel 1 zur Bestimmung der im Reaktionsgemisch entstandenen F1+2-Menge benutzt.

Zusätzlich erfolgte eine Kalibration des erfindungsgemäßen F1+2 Generierungs-Assays unter Verwendung eines Plasmakalibratormaterials mit definiertem Prothrombingehalt (% der Norm) (Innovance ETP Standard, Siemens Healthcare Diagnostics Products GmbH, Deutschland). Der Standard wurde zur Ermittlung der Kalibrationskurve (FIG. 2) mit OVB-Puffer verdünnt. Der oberste Kalibrationspunkt bei 196% wurde nicht gemessen, sondern extrapoliert.

Zum Vergleich mit dem Endogenen Thrombinpotenzial (ETP) wurden die Plasmaproben ferner mit dem INNOVANCE ETP Assay (Siemens Healthcare Diagnostics Products GmbH, Deutschland) gemessen. Bei dieser bekannten Methode zur ETP-Bestimmung werden der Probe ein Gerinnungsaktivator (Innovin und Calciumionen), ein chromogenes Thrombinsubstrat und ein Fibrininhibitor zugesetzt, und es wird über einen Zeitraum von etwa 20 Minuten die Umsatzkinetik eines Thrombinsubstrats photometrisch bestimmt. Die Bestimmung des ETP erfolgt anhand der Parameter Cmax (maximale Reaktionsgeschwindigkeit) und AUC (area under the curve) der gemessenen Umsatzkinetik. Es wird eine Kalibrationskurve erstellt unter Verwendung des Innovance ETP Standards (Siemens Healthcare Diagnostics Products GmbH, Deutschland), so dass die Ergebnisse die Einheit % der Norm erhalten. Die automatische Abarbeitung des ETP-Testes erfolgte auf dem BCS XP Analysesystem (Siemens Healthcare Diagnostics Products GmbH, Deutschland).

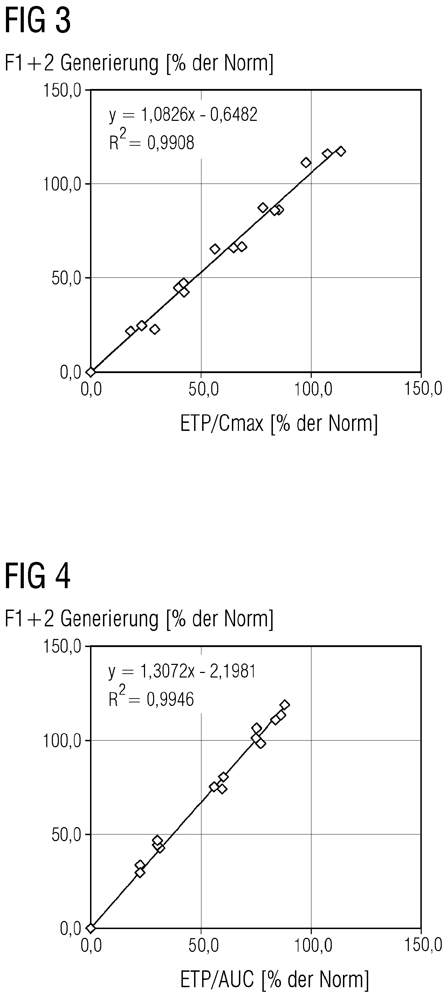

Für den Zweck des Vergleichs von F1+2 Generierung und Endogenem Thrombinpotenzial wurden Plasmaproben hergestellt, indem ein Normalplasmapool (Mischung aus Plasmen mehrerer gesunder Spender) mit einem Mangelplasmapool (Mischung aus Plasmen mehrerer gesunder Spender, in der mittels AlOH-Absorption ein starker Mangel an mehreren Gerinnungsfaktoren hergestellt worden ist) in mehreren Stufen verdünnt wurde FIG. 3 und 4 zeigen, dass der Verlust an Gerinnungsfaktoren sich beim ETP- und beim F1+2- Generierungstest sich in gleicher Weise in einem Abfall der % der Norm-Werte äußert.

Dies zeigt, dass der F1+2 Generierungstest, dessen Messprinzip und Auswertung wesentlich einfacher ist als das Messprinzip und die Auswertung des Endogenen Thrombinpotenzials, in gleicher Weise auf den Verlust von Gerinnungsfaktoren reagiert und somit in gleicher Weise eine antikoagulatorische Situation erkennt wie der Thrombingenerierungstest.

### BEISPIEL 3: Vergleichende Bestimmung von Einzelfaktor-Defizienzen mittels Messung des F1+2 Generierungspotenzials und Messung des endogenen Thrombinpotenzials (ETP)

Zur Ermittlung der Abhängigkeit des F1+2 Generierungspotenzials von einzelnen Gerinnungsfaktoren wurden Einzelfaktor-defiziente Proben hergestellt. Hierzu wurde ein Normalplasmapool mit verschiedenen Faktor-Mangelplasmen (Faktor II-, VII-, IX- und X-Mangelplasmen) in verschiedenen Stufen verdünnt. Diese Proben wurden sowohl mit dem Thrombingenerierungstest INNOVANCE ETP wie auch mit dem erfindungsgemäßen F1+2 Generierungstest wie in Beispiel 2 gemessen.

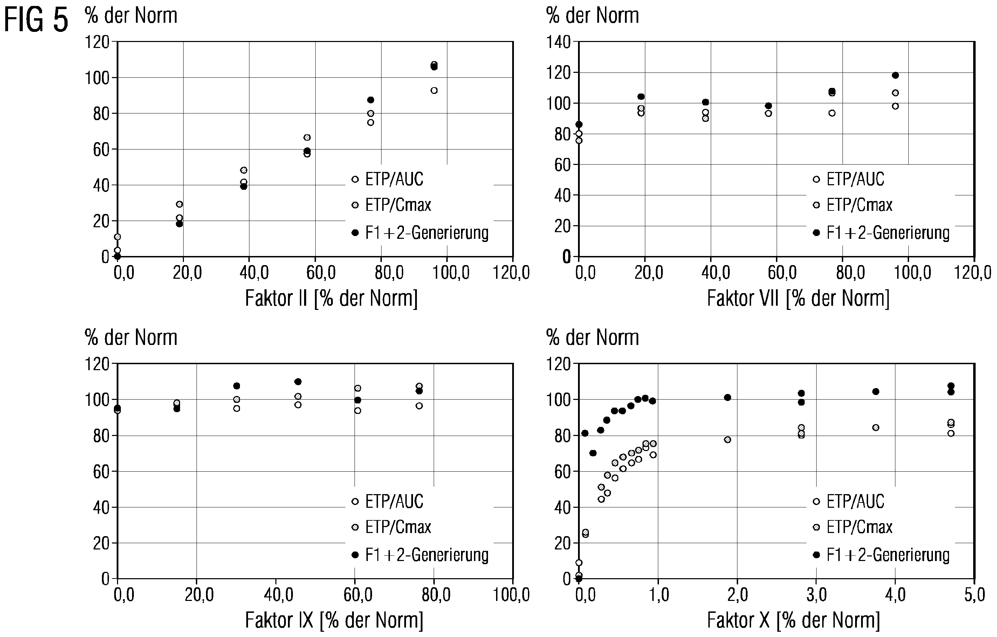

Generell zeigt sich ein Abfall der Werte in Abhängigkeit von der Faktor-Konzentration in ähnlicher Weise beim Thrombingenerierungstest wie auch beim F1+2-Generierungstest (FIG. 5). Die Abhängigkeit von der Faktor II-Aktivität ist in beiden Fällen sehr stark und nahezu linear. Ein Faktor VII-Mangel wirkt sich nur schwach aus und ist erst unterhalb von 20% der Norm bemerkbar. Ein Faktor-IX Mangel zeigt überhaupt keine Auswirkungen. Der Faktor X-Mangel wirkt sich erst unterhalb von 1% der Norm aus, wobei in reinem Mangelplasma sowohl die Thrombin- wie auch die F1+2-Generierung komplett verhindert wird.

Der F1+2-Generierungstest zeigt folglich gleichermaßen die Auswirkungen von Faktorenmängeln an wie der Thrombingenerierungstest.

### BEISPIEL 4: Feststellung eines antikoagulatorischen Status des Blutgerinnungssystems durch Erfassung der Stärke der Antikoagulation eines Faktor Xa-Inhibitors

Das als Gerinnungsaktivator wirkende TF/Lipid-Reagenz wurde folgendermaßen hergestellt: Innovin Reagenz (siehe Beispiel 1) wurde 1:98,6 mit Owrens Veronal Buffer verdünnt. Zu 1493,3 µL dieser Verdünnung wurden 6,7 µL einer Phospholipid-Suspension (68% 1,2-Dioleoyl-sn-Glycero-3-Phosphocholin, 32% 1-Palmitoyl-2-Oleoyl-sn-Glycero-3-Phosphoserin, zusammen 11,4 g/L) zugegeben.

Mit diesem modifizierten Gerinnungsaktivator wurde der F1+2-Generierungstest wie in Beispiel 2 beschrieben durchgeführt.

Zur Herstellung von antikoagulierten Proben wurde verfahren wie bei der Erstellung der Kalibrationskurve des INNOVANCE Anti-Xa Rivaroxaban Assay (Siemens Healthcare Diagnostics Products GmbH, Deutschland). Ein Standard 0 (= Normalplasmapool ohne Rivaroxaban) und ein Standard 1 (Normalplasmapool mit 410 ng/mL Rivaroxaban) wurden in verschiedenen Verhältnissen gemischt.

Die Proben wurden mit dem INNOVANCE Anti-Xa Rivaroxaban Assay, einem chromogenen anti-FXa-Test zur Bestimmung der Konzentration von Rivaroxaban, und dem erfindungsgemäßen F1+2-Generierungstest gemessen.

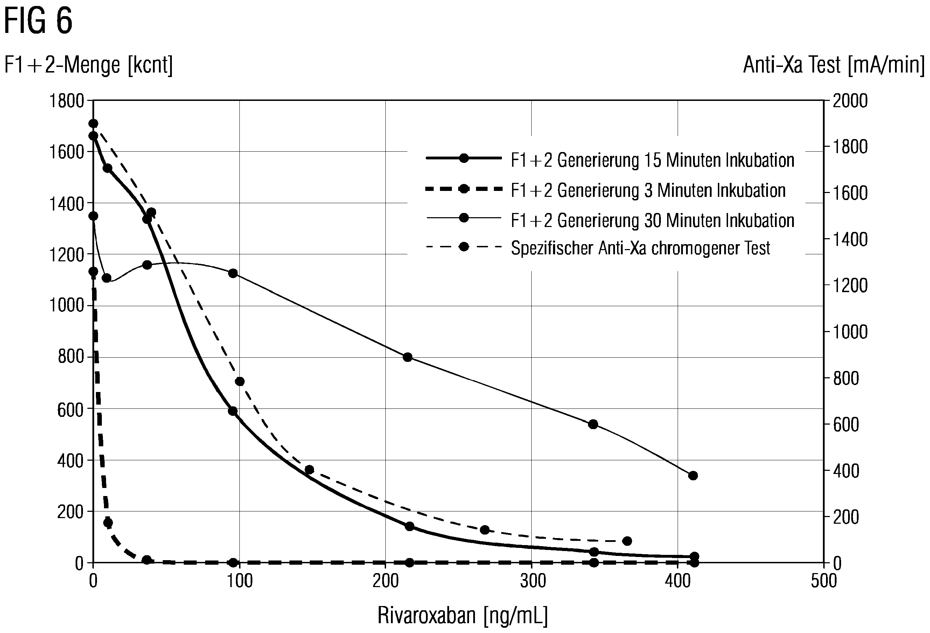

Es zeigt sich beim F1+2-Generierungstest bei einer Inkubationszeit von 15 Minuten nach Zugabe von CaCl₂ eine sehr ähnliche Abhängigkeit der Signale (kcnt) von der Rivaroxaban-Konzentration wie bei dem spezifischen Anti-Xa Rivaroxaban-Test (mA-Signal) (FIG. 6). Der F1+2 Generierungstest kann also gleichermaßen zur Quantifizierung von Antikoagulantien verwendet werden. Vorteilhafterweise kann der Test sehr einfach durch eine Verkürzung der Inkubationszeit eine höhere Sensitivität erhalten oder durch eine Verlängerung der Inkubationszeit einen weiteren Messbereich erhalten. Bei einer Inkubationszeit von 3 Minuten (nach CaCl₂-Zugabe) reagiert der Test extrem empfindlich auf eine Erhöhung der Rivaroxaban-Konzentration von 0 auf 10 ng/mL. Bei einer Inkubationszeit von 30 Minuten (nach CaCl₂-Zugabe) kann der Messbereich auf über 400 ng/mL ausgedehnt werden. Eine solche Änderung der Inkubationszeit kann automatisch von dem Analysensystem als Wiederholungmessung durchgeführt werden, wenn das initiale Messergebnis kleiner oder größer als der Messbereich ist.

### BEISPIEL 5: Feststellung eines antikoagulatorischen Status des Blutgerinnungssystems durch Erfassung der Stärke der Antikoagulation eines Faktor IIa-Inhibitors

Das als Gerinnungsaktivator wirkende TF/Lipid-Reagenz wurde wie in Beispiel 4 beschrieben hergestellt, und damit wurde der F1+2-Generierungstest wie in Beispiel 2 beschrieben durchgeführt.

Zur Herstellung von antikoagulierten Proben wurde verfahren wie bei der Erstellung der Kalibrationskurve des INNOVANCE DTI Assay (Siemens Healthcare Diagnostics Products GmbH, Deutschland). Ein Standard 0 (= Normalplasmapool ohne Dabigatran) und ein Standard 1 (Normalplasmapool mit 548 ng/mL Dabigatran) wurden in verschiedenen Verhältnissen gemischt.

Die Proben wurden mit dem INNOVANCE DTI Assay, einem chromogenen anti-FIIa-Test zur Bestimmung der Konzentration von Dabigatran, gemessen.

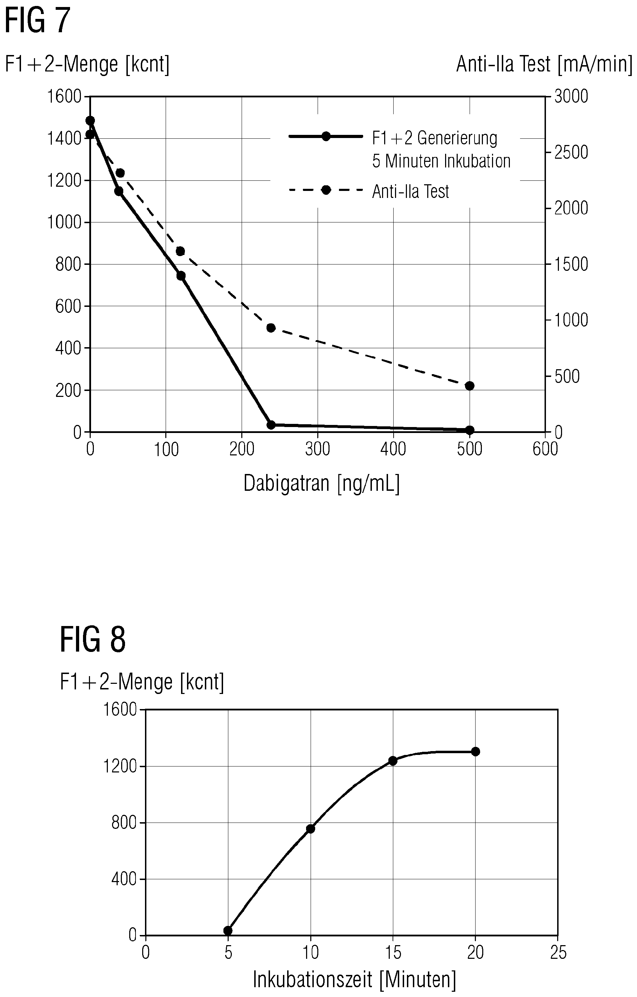

Es zeigt sich beim F1+2-Generierungstest bei einer Inkubationszeit von 5 Minuten nach Zugabe von CaCl₂ eine sehr ähnliche Abhängigkeit der Signale (kcnt) von der Dabigatran-Konzentration wie bei dem spezifischen Anti-IIa Dabigatran-Test (mA/min-Signal) (FIG. 7). Der F1+2 Generierungstest kann also gleichermaßen zur Quantifizierung von Antikoagulantien verwendet werden. Vorteilhafterweise kann der Test sehr einfach durch eine Verkürzung der Inkubationszeit eine höhere Sensitivität erhalten oder durch eine Verlängerung der Inkubationszeit (nach CaCl₂-Zugabe) einen weiteren Messbereich erhalten. FIG. 8 zeigt die Steigerung der Signalhöhe im F1+2-Generierungstest bei einer Dabigatran-Konzentration von 120 ng/mL durch eine Erhöhung der Inkubationszeit. Eine solche Änderung der Inkubationszeit kann automatisch von dem Analysensystem als Wiederholungmessung durchgeführt werden, wenn das initiale Messergebnis kleiner oder größer als der Messbereich ist.

### BEISPIEL 6: Verkürzung der Inkubationszeit beim F1+2 Generierungstest

Die vergleichende Bestimmung der in vitro-generierten F1+2-Menge und des ETP's erfolgte wie in Beispiel 2 beschrieben, wobei im F1+2-Generierungstest anstelle einer Inkubation von 20 Minuten nach der Zugabe von CaCl₂ nur eine Inkubation von 5 Minuten durchgeführt wurde. Die Kalibration sowie die Herstellung und Messung der Proben (Proben mit zunehmendem Gerinnungsfaktormangel in % der Norm) erfolgte wie in Beispiel 2 beschrieben.

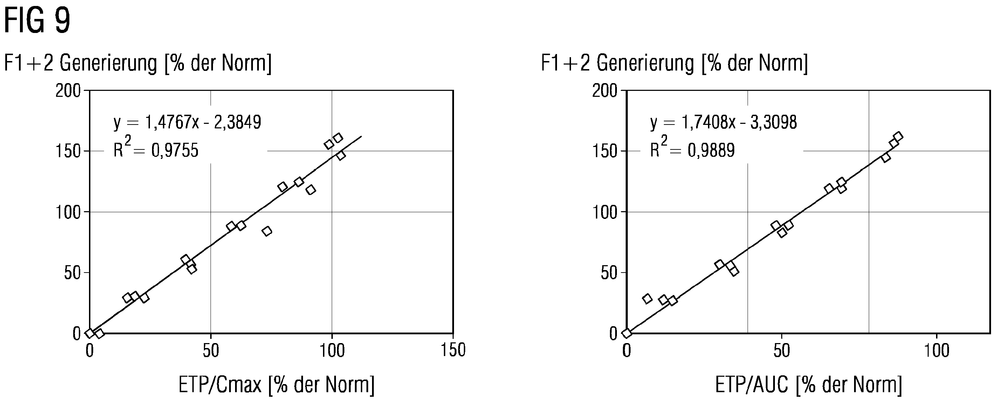

Die Korrelation der Ergebnisse des F1+2-Generierungstests mit 5 Minuten Inkubation mit dem klassischen ETP-Test bleibt linear (siehe FIG. 9). Die Steigung der Geraden (deutlich größer als 1) deutet darauf hin, dass eine spezifische Standardisierung notwendig ist. Da die Verringerung der Ergebnisse den gleichen linearen Zusammenhang mit der Verringerung der Gerinnungsfaktoren zeigt, ist die Verringerung der Inkubationzeit möglich. Damit verkürzt sich die Gesamttestdauer des F1+2-Generierungstests um 15 Minuten auf dann 7,5 Minuten, was einen erheblichen Vorteil gegenüber dem ETP-Test aus dem Stand der Technik darstellt.

### BEISPIEL 7: Feststellung eines prokoagulatorischen Status des Blutgerinnungssystems durch Erfassung einer erhöhten Thrombozytenaktivierung mittels Messung des F1+2 Generierungspotenzials

Die Detektion eines hyper- oder hypokoagulatorischen Zustands, welcher seine Ursache in einer gestörten Thrombin- bzw. F1+2-Generierungs-Funktionalität von Blutzellen hat, ist nur möglich, wenn diese Blutzellen in der Probe präsent sind. Zur Erfassung von Störungen der Funktionalität der Thrombozyten muss entweder PRP oder Vollblut als Probe verwendet werden. Zur Erfassung von Störungen der anderen Blutzellen muss Vollblut als Probe verwendet werden.

Ein hyperkoagulatorischer Zustand der Probe (Vollblut, PRP oder Plasma) wurde durch eine Präaktivierung der Thrombozyten mit Kollagen erreicht. Dazu wurden 900 µL Probe mit 100 µL Kollagenlösung (20 µg/mL) bzw. zur Kontrolle mit 100 µL OVB-Puffer vermischt, und die Mischung wurde für 20 Minuten bei 37 °C im Wasserbad inkubiert.

Um den Einfluss der Blutzellen sensitiv erkennen zu können, wurde das Innovin-Reagenz, welches Tissue Factor und Lipide enthält, 1:780 mit OVB-Puffer verdünnt.

Die präaktivierten Proben wurden mit den Komponenten des INNOVANCE ETP Assay (Siemens Healthcare Diagnostics Products GmbH, Deutschland) wie folgt gemischt:
108 µL Probe
+ 40 µL ETP Puffer
+ 35 µL ETP Reagenz
150 Sekunden Inkubation
+ 10 µL Innovin Reagenz (1:780 vorverdünnt mit OVB)
+ 14 µL ETP CaCl₂
30 Minuten Inkubation bei 37°C
1:100-Verdünnung des Reaktionsgemischs mit OVB-Puffer.

5 µL des verdünnten Reaktionsgemischs wurden wie in Beispiel 1 zur Bestimmung der im Reaktionsgemisch entstandenen F1+2-Menge benutzt.

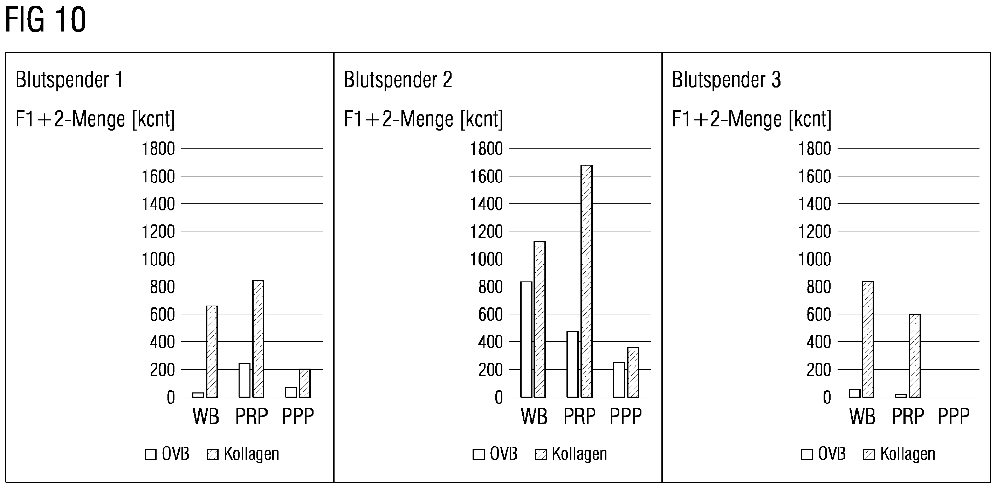

Untersucht wurden drei anscheinend gesunde Blutspender. Die Präaktivierung der Thrombozyten konnte bei Verwendung von PRP oder Vollblut als Probe durch die stark erhöhte F1+2 Generierung im Vergleich zu nicht präaktivierten Thrombozyten (Zugabe von OVB-Puffer anstelle von Kollagen) im Vollblut und PRP nachgewiesen werden (siehe FIG. 10). Somit kann mittels Messung des F1+2-Generierungspotenzials in Vollblut oder PRP eine zellulär bedingte Hyperkoagulabilität und damit ein prokoagulatorischer Status des Blutgerinnungssystems nachgewiesen werden.

Der Blutspender 2 fällt durch ein besonders hohes F1+2 Generierungspotenzial auf, welches bereits ohne Kollagen-Aktivierung der Plättchen und auch bei Plasma als Probe offensichtlich ist. Dies weist darauf hin, dass bei diesem Spender in vivo ein prokoagulatorischer Status des Blutgerinnungssystems vorliegt.

### BEISPIEL 8: Feststellung eines antikoagulatorischen Status des Blutgerinnungssystems durch Erfassung der antikoagulatorischen Wirkung einer verminderten Thrombozytenzahl

Es wurden PRP- und PPP- Proben von drei offensichtlich gesunden Blutspendern hergestellt und die Thrombozytenzahl bestimmt.

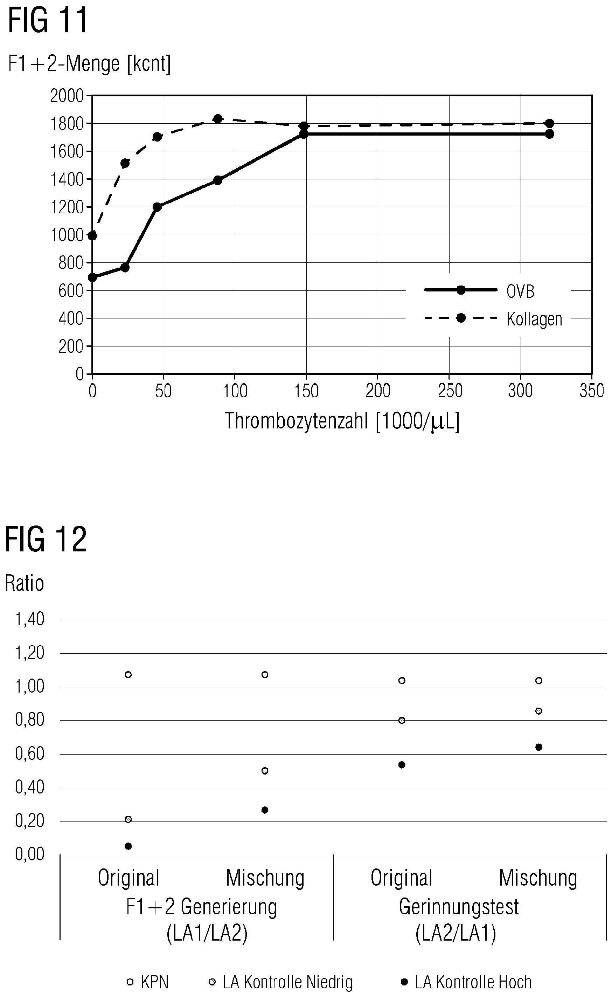

Es erfolgte eine Messung des F1+2-Generierungspotenzials mit PRP als Probe und mit und ohne Präaktivierung durch Kollagenzugabe wie in Beispiel 7 beschrieben, wobei die Kollagenlösung hier eine Konzentration von 80 µg/mL aufweist. Die Ergebnisse der Proben der drei Blutspender wurden gemittelt. Ab einer Thrombozytenzahl < 100.000/µL verringert sich das F1+2 Generierungspotenzial, wenn die Thrombozyten nicht präaktiviert wurden. Bei einer Präaktivierung der Thrombozyten verringert sich das F1+2-Generierungspotenzial ab einer Thrombozytenzahl < 50.000/µL (siehe FIG. 11).

Die Messung des F1+2 Generierungspotenzial zeigt folglich einen antikoagulatorischen Status des Blutgerinnungssystems an, der durch eine Thrombozytopenie bedingt ist. Die Ergebnisse der F1+2 Generierung spiegeln die Situation in vivo wider, bei der eine Thrombozytopenie sich weniger stark auswirkt, wenn die vorhandenen Thrombozyten besonders aktiv sind.

### BEISPIEL 9: Feststellung eines prokoagulatorischen Status des Blutgerinnungssystems durch Erfassung der prokoagulatorischen Wirkung von Lupus Antikoagulanz

Der am häufigsten benutzte Test zur Bestimmung von Lupus Antikoagulanz (LA) ist der sogenannte DRVVT-Test (Dilute Russel Viper Venom Test). Bei diesem Test wird mit RVV als Gerinnungsaktivator die Gerinnung ausgelöst. Zuerst wird ein Test durchgeführt, bei dem im Gerinnungsaktivatorreagenz nur sehr wenig Phospholipide enthalten sind (LA1-Test). Liegt Lupus Antikoagulanz vor, verlängert sich die Gerinnungszeit im LA1-Test. Dann wird ein zweiter Test (LA2) durchgeführt unter Benutzung eines Gerinnungsaktivatorreagenzes, das eine höhere Phospholipid-Konzentration aufweist. Der LA2-Test zeigt auch dann eine annähernd normale Gerinnungszeit, wenn Lupus Antikoagulanz vorliegt. Ein LA2/LA1 Ratio der beiden Teste ist annähernd 1, wenn eine Normalprobe gemessen wird und sinkt unter 1, wenn Lupus Antikoagulanz vorliegt.

Der Lupus Antikoagulanz-Test wurde in herkömmlicher Weise durchgeführt, jedoch mit dem Unterschied, dass anstelle der Messung der Gerinnungszeit die im Reaktionsgemisch entstandene F1+2-Menge bestimmt wurde (siehe Beispiel 1).

Durchführung des LA1-Tests:
100 µL Probe
240 Sekunden Inkubation bei 37 °C
+ 100 µL LA1-Reagenz
80 Sekunden Inkubation bei 37 °C
1:50-Verdünnung Reaktionsgemischs mit OVB-Puffer.

Durchführung des LA2-Tests:
100 µL Probe
240 Sekunden Inkubation bei 37 °C
+ 100 µL LA2-Reagenz
80 Sekunden Inkubation bei 37 °C
1:50-Verdünnung Reaktionsgemischs mit OVB-Puffer.

Es wurden jeweils 5 µL der verdünnten Reaktionsgemische zur Bestimmung der entstandenen F1+2-Menge benutzt (siehe Beispiel 1).

Es wurde eine Normalplasmaprobe ohne Lupus Antikoagulanz (KPN), eine Probe mit hoher LA-Konzentration (LA-Kontrolle hoch) und eine Probe mit geringer LA-Konzentration (LA-Kontrolle niedrig) getestet sowie eine 1:1-Verdünnung der LA-Kontrollen hoch und niedrig mit dem Normalplasma.

In FIG. 12 werden die LA2/LA1-Ratios des Gerinnungstests mit den LA1/LA2-Ratios des F1+2 Generierungstests verglichen. Für das Normalplasma liegen alle Ratios nahe 1. Die Probe mit niedrigem Lupus Antikoagulanz-Gehalt (LA-Kontrolle niedrig) weist ein LA1/LA2-Ratio im F1+2-Generierungstest auf (0,21), das wesentlich geringer ist als das LA2/LA1-Ratio im Gerinnungstest (0,80). Der F1+2 Generierungstest reagiert also wesentlich sensitiver auf einen schwach pathologischen Lupus Antikoagulanz-Gehalt als der Gerinnungstest.

Bei der Lupus Antikoagulanz-Testung wird die Probe mit einem Normalplasma verdünnt, wenn Faktorenmängel als Ursache für pathologische Werte ausgeschlossen werden sollen. Bei der 1:1-Mischung mit Normalplasma (KPN) sinkt bei der LA-Kontrolle niedrig bei dem Gerinnungstest das Ratio nur noch auf 0,86 ab, während beim F1+2-Generierungstest das Ratio immer noch auf 0,50 abgesunken ist. Auch in einer 1:1-Mischung mit normalem Plasma kann folglich ein schwach ausgeprägtes Lupus Antikoagulanz mittels Messung der F1+2-Generierung wesentlich besser erkannt werden.

### BEISPIEL 10: Feststellung eines antikoagulatorischen Status des Blutgerinnungssystems durch Erfassung der Stärke der Antikoagulation eines Thrombozytenhemmers

Bei Patienten mit erhöhtem Thromboserisiko ist eine Hemmung der Thrombozyten als prophylaktische Therapie indiziert. Ein solche Hemmung erfolgte in diesem Beispiel mit Prostaglandin E1- eine Substanz, die bekannt ist für die Hemmung der Thrombozytenaggregation.

Vollblut von 3 normalen und gesunden Blutspendern wurde mit 5% (v/v) einer Prostaglandin E1 Lösung versetzt (Endkonzentration in der Probe 20 µmol/L) und 20 Minuten bei Raumtemperatur inkubiert. Anschließend erfolgte die Aktivierung durch Kollagen und weitere Behandlung und Testung der Proben wie in Beispiel 7 beschrieben.

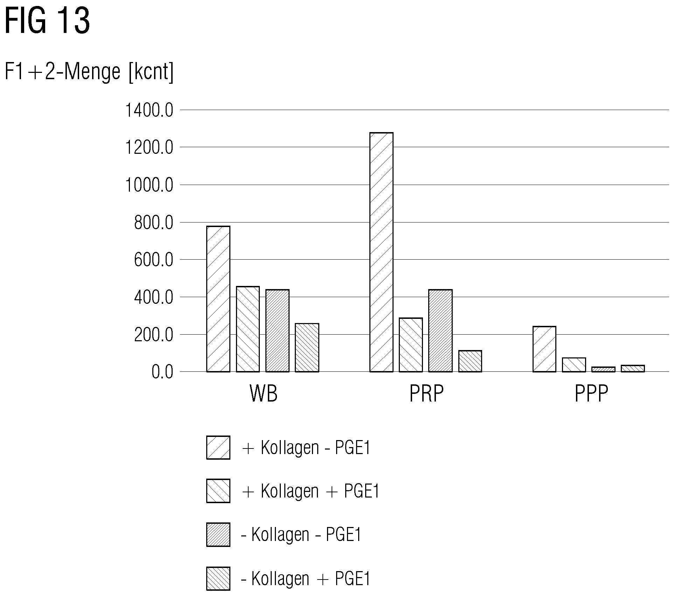

FIG. 13 zeigt die F1+2 Generierungspotenziale von Proben mit und ohne PGE1-Vorbehandlung und mit und ohne nachfolgender Kollagen-Aktiverung. Die antikoagulatorische Wirkung des Thrombozytenhemmers PGE1 zeigt sich sowohl ohne als auch mit Aktivierung der Thrombozyten durch Kollagen als verminderte F1+2 Generierung. Der erfindungsgemäße Test kann zur Therapiekontrolle von Thrombozytenhemmern verwendet werden.

### BEISPIEL 11: Hochsensitive Bestimmung der Faktor VIII-Aktivität

Durch Mischung eines Normalplasmapools mit bekannter Faktor VIII-Aktivität (Kontroll Plasma N) mit Owrens Veronal Buffer wurden 3 Proben mit bekannter Faktor VIII-Aktivität hergestellt. Diese Proben werden wie folgt mit Reagenzien gemischt. Alle Reagenzien werden von Siemens Healthcare Diagnostics Products GmbH, Deutschland, vertrieben.
2 µL Probe
+ 18 µL Puffer
10 Sekunden Inkubation
+ 15 µL Faktor VIII Mangelplasma (< 1% Faktor VIII)
120 Sekunden Inkubation
+ 30 µL Actin FS Reagenz (Gerinnungsaktivator)
180 Sekunden Inkubation
+ 40 µL CaCl₂-Lösung
120 Sekunden Inkubation
+ 15 µL H₂O

5 µL des verdünnten Reaktionsgemischs wurden wie in Beispiel 1 zur Bestimmung der im Reaktionsgemisch entstandenen F1+2-Menge benutzt.

Nach einer weiteren Inkubation des Reaktionsansatzes für einen Zeitraum von 6 Minuten wurde das Chemilumineszenzsignal in der willkürlichen Einheit kilocounts (kcnt) gemessen.

### Ergebnisse:

| | |
|---|---|
| Probe 1 | 0 % Faktor VIII = 1876 kcnt |
| Probe 2 | 0,5 % Faktor VIII = 2469 kcnt |
| Probe 3 | 1,0 % Faktor VIII = 2779 kcnt |
| Probe 4 | 1,5 % Faktor VIII = 2901 kcnt |

Der F1+2-Generierungstest erlaubt einen hochsensitiven Faktor VIII-Aktivitätstest mit großen Signaländerungen im Bereich zwischen 0 und 1,5 % Faktor VIII-Aktivität.

## Patentansprüche

1. Verfahren zur Feststellung des Status des Blutgerinnungssystems eines Individuums, das Verfahren umfassend die Schritte:
a. Bereitstellen eines Reaktionsgemisches durch Zugabe eines Gerinnungsaktivators zu einer Probe des Individuums und
b. Inkubation des Reaktionsgemisches,
**dadurch gekennzeichnet, dass** das Verfahren ferner die folgenden Schritte umfasst:
c. quantitative Bestimmung der F1+2-Menge in dem Reaktionsgemisch und
d. Feststellung des Status des Blutgerinnungssystems durch Vergleichen der so gemessenen F1+2-Menge in dem Reaktionsgemisch mit einem Referenzwert oder einem Referenzwertbereich, der einen normalen Status des Blutgerinnungssystems eines gesunden Individuums repräsentiert.

2. Verfahren gemäß Anspruch 1, wobei ein prokoagulatorischer Status des Blutgerinnungssystems des Individuums festgestellt wird, wenn die in Schritt c) gemessene F1+2-Menge größer ist als der Referenzwert oder der Referenzwertbereich, der einen normalen Status des Blutgerinnungssystems eines gesunden Individuums repräsentiert.

3. Verfahren gemäß Anspruch 1, wobei ein antikoagulatorischer Status des Blutgerinnungssystems des Individuums festgestellt wird, wenn die in Schritt c) gemessene F1+2-Menge kleiner ist als der Referenzwert oder der Referenzwertbereich, der einen normalen Status des Blutgerinnungssystems eines gesunden Individuums repräsentiert.

4. Verfahren gemäß Anspruch 1, wobei das Reaktionsgemisch nach der Inkubation in Schritt b) durch Zugabe eines Verdünnungsmediums verdünnt wird und die quantitative Bestimmung der F1+2-Menge in Schritt c) in dem so verdünnten Reaktionsgemisch durchgeführt wird.

5. Verfahren gemäß einem der Ansprüche 1 bis 4, wobei die quantitative Bestimmung der F1+2-Menge in dem Reaktionsgemisch oder in dem verdünnten Reaktionsgemisch in Schritt c) mit einem F1+2-spezifischen Immunoassay durchgeführt wird.

6. Verfahren gemäß Anspruch 5, wobei der F1+2-spezifische Immunoassay die Verwendung eines ersten Antikörpers mit Spezifität für F1+2 und die Verwendung eines zweiten, F1+2-bindenden Antikörpers oder eines zweiten Antikörpers mit Spezifität für den Immunkomplex bestehend aus F1+2 und dem ersten Antikörper umfasst.

7. Verfahren gemäß Anspruch 6, wobei der erste und der zweite Antikörper jeweils mit einer partikulären Festphase assoziiert sind und die quantitative Bestimmung der F1+2-Menge die folgenden Schritte umfasst:
i. Vermischen des Reaktionsgemisches mit den partikulären Festphasen, die mit dem ersten und zweiten Antikörper assoziiert sind und
ii. Messen der Agglutination der partikulären Festphasen im Reaktionsgemisch.

8. Verfahren gemäß Anspruch 7, wobei die Agglutination der partikulären Festphasen in dem Reaktionsgemisch photometrisch gemessen wird.

9. Verfahren gemäß Anspruch 6, wobei der erste Antikörper mit einer ersten partikulären Festphase assoziiert ist und der zweite Antikörper mit einer zweiten partikulären Festphase assoziiert ist und wobei die erste partikuläre Festphase mit einer ersten Komponente eines signalbildenden Systems assoziiert ist und die zweite partikuläre Festphase mit einer zweiten Komponente des signalbildenden Systems assoziiert ist, und wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden und die Agglutination der partikulären Festphasen in dem Reaktionsgemisch anhand des entstandenen Signals gemessen wird.

10. Verfahren gemäß Anspruch 9, wobei die erste Komponente des signalbildenden Systems ein chemilumineszierendes Agens ist und die zweite Komponente des signalbildenden Systems ein Photosensitizer ist oder umgekehrt und wobei die Chemilumineszenz in dem Reaktionsgemisch gemessen wird.

11. Verfahren gemäß Anspruch 1, wobei das Reaktionsgemisch in Schritt b) für einen Zeitraum von 5 Sekunden bis 60 Minuten, vorzugsweise von 5 bis 20 Minuten inkubiert wird.

12. Verfahren gemäß Anspruch 1, wobei der Gerinnungsaktivator ein Aktivator des plasmatischen Gerinnungssystems ist, vorzugsweise aus der Gruppe Thromboplastin, Faktor IIa, Faktor VIIa, Faktor IXa, Faktor Xa, Faktor XIa, Faktor XIIa, Schlangengifte, negativ geladene Phospholipide, Calciumionen, Gewebefaktor, Silica, Kaolin, Ellagsäure, Celite und Polyphosphate.

13. Verfahren gemäß Anspruch 1, wobei der Gerinnungsaktivator ein Plättchenaktivator ist, vorzugsweise aus der Gruppe ADP, Epinephrin, Kollagen, Thrombin-Rezeptor aktivierendes Peptid (TRAP), Thromboxan A2 nachahmendes U46619, Arachidonsäure, Ristocetin, Thrombin, von Willebrand Faktor, Kollagen-bezogenes Peptid (GPVI-Stimulation), Convulxin, Kalzium Ionophor A23187 und Phorbol 12-Myristat 13 Azetat.

14. Verfahren gemäß Anspruch 1, wobei der Gerinnungsaktivator ein chemischer oder physikalischer Faktor ist, welcher die prokoagulatorische Wirkung von Erythrozyten verstärkt.

15. Verfahren gemäß Anspruch 1, wobei der Gerinnungsaktivator ein Leukozyten-aktivierender Faktor ist.

## Claims

1. A method for establishing an individual's blood coagulation system status, the method comprising the following steps:
a. providing a reaction mixture by adding a coagulation activator to a sample from the individual and
b. incubating the reaction mixture,
**characterized in that** the method additionally comprises the following steps:
c. quantitative determination of the amount of F1+2 in the reaction mixture and
d. establishing the blood coagulation system status by comparing the amount of F1+2 thus measured in the reaction mixture with a reference value or reference value range that represents the normal blood coagulation system status of a healthy individual.

2. The method as claimed in claim 1, wherein a procoagulatory status of an individual's blood coagulation system is established when the amount of F1+2 measured in step c) is above the reference value or reference value range that represents a normal blood coagulation system status of a healthy individual.

3. The method as claimed in claim 1, wherein an anticoagulatory status of an individual's blood coagulation system is established when the amount of F1+2 measured in step c) is below the reference value or reference value range that represents a normal blood coagulation system status of a healthy individual.

4. The method as claimed in claim 1, wherein the reaction mixture is diluted after the incubation in step b) and the quantitative determination of the amount of F1+2 in step c) is carried out in the reaction mixture thus diluted.

5. The method as claimed in any of claims 1 to 4, wherein the quantitative determination of the amount of F1+2 in the reaction mixture or in the diluted reaction mixture in step c) is carried out using a F1+2-specific immunoassay.

6. The method as claimed in claim 5, wherein the F1+2-specific immunoassay comprises the use of a first antibody having specificity for F1+2 and the use of a second, F1+2- binding antibody or of a second antibody having specificity for the immune complex consisting of F1+2 and the first antibody.

7. The method as claimed in claim 6, wherein the first and second antibodies are each associated with a particulate solid phase and the quantitative determination of the amount of F1+2 comprises the following steps:
i. mixing the reaction mixture with the particulate solid phases associated with the first and second antibodies and
ii. measuring the agglutination of the particulate solid phases in the reaction mixture.

8. The method as claimed in claim 7, wherein the agglutination of the particulate solid phases in the reaction mixture is measured photometrically.

9. The method as claimed in claim 6, wherein the first antibody is associated with a first particulate solid phase and the second antibody is associated with a second particulate solid phase and wherein the first particulate solid phase is associated with a first component of a signal-forming system and the second particulate solid phase is associated with a second component of a signal-forming system, and wherein the first and the second component of the signal-forming system interact with one another such that this produces a detectable signal when the first and the second component of the signal-forming system are brought into spatial proximity and the agglutination of the particulate solid phases in the reaction mixture is measured on the basis of the signal produced.

10. The method as claimed in claim 9, wherein the first component of the signal-forming system is a chemiluminescent agent and the second component of the signal-forming system is a photosensitizer or vice-versa and wherein the chemiluminescence in the reaction mixture is measured.

11. The method as claimed in claim 1, wherein the reaction mixture in step b) is incubated for a period of from 5 seconds to 60 minutes, preferably from 5 to 20 minutes.

12. The method as claimed in claim 1, wherein the coagulation activator is an activator of the plasmatic coagulation system, preferably from the group comprising thromboplastin, factor IIa, factor VIIa, factor IXa, factor Xa, factor XIa, factor XIIa, snake venoms, negatively charged phospholipids, calcium ions, tissue factor, silica, kaolin, ellagic acid, Celite, and polyphosphates.

13. The method as claimed in claim 1, wherein the coagulation activator is a platelet activator, preferably from the group comprising ADP, epinephrine, collagen, thrombin-receptor-activating peptide (TRAP), thromboxane A2 mimic U46619, arachidonic acid, ristocetin, thrombin, von Willebrand factor, collagen-related peptide (GPVI stimulation), convulxin, calcium ionophore A23187, and phorbol 12-myristate 13-acetate.

14. The method as claimed in claim 1, wherein the coagulation activator is a chemical or physical factor that heightens the procoagulatory effect of erythrocytes.

15. The method as claimed in claim 1, wherein the coagulation activator is a leukocyte-activating factor.

## Revendications

1. Procédé de détermination de l'état du système de coagulation sanguine d'un individu, le procédé comprenant les stades :
a. se procurer un mélange réactionnel par addition d'un activateur de coagulation à un échantillon d'individu et
b. incubation du mélange réactionnel,
**caractérisé en ce que** le procédé comprend en outre les stades suivants :
c. détermination quantitative de la quantité F1+2 dans le mélange réactionnel et
d. détermination de l'état du système de coagulation sanguine par comparaison de la quantité F1+2 ainsi mesurée dans le mélange réactionnel à une valeur de référence ou à une plage de valeurs de référence, qui représente un état normal du système de coagulation sanguine d'un individu sain.

2. Procédé suivant la revendication 1, dans lequel on détermine un état procoagulateur du système de coagulation sanguine de l'individu, si la quantité F1+2 mesurée dans le stade c) est plus grande que la valeur de référence ou que la plage de valeurs de référence, qui représente un état normal du système de coagulation sanguine d'un individu sain.

3. Procédé suivant la revendication 1, dans lequel on détermine un état anticoagulateur du système de coagulation sanguine de l'individu, si la quantité F1+2 mesurée dans le stade c) est plus petite que la valeur de référence ou que la plage de valeurs de référence, qui représente un état normal du système de coagulation sanguine d'un individu sain.

4. Procédé suivant la revendication 1, dans lequel on dilue le mélange réactionnel après l'incubation dans le stade b) par addition d'un milieu de dilution et on effectue la détermination quantitative de la quantité F1+2 dans le stade c) dans le mélange réactionnel ainsi dilué.

5. Procédé suivant l'une des revendications 1 à 4, dans lequel on effectue la détermination quantitative de la quantité F1+2 dans le mélange réactionnel ou dans le mélange réactionnel dilué dans le stade c) par un immunodosage spécifique à F1+2.

6. Procédé suivant la revendication 5, dans lequel l'immunodosage spécifique à F1+2 comprend l'utilisation d'un premier anticorps ayant une spécificité pour F1+2 et l'utilisation d'un deuxième anticorps fixant F1+2 ou d'un deuxième anticorps ayant une spécificité pour l'immunocomplexe constitué de F1+2 et du premier anticorps.

7. Procédé suivant la revendication 6, dans lequel le premier et le deuxième anticorps sont associés respectivement à une phase solide en particules et la détermination quantitative de la quantité F1+2 comprend les stades suivants :
i. mélange du mélange réactionnel aux phases solides en particules, qui sont associées au premier et au deuxième anticorps, et
ii. mesure de l'agglutination des phases solides en particules dans le mélange réactionnel.

8. Procédé suivant la revendication 7, dans lequel on mesure photométriquement l'agglutination des phases solides en particules dans le mélange réactionnel.

9. Procédé suivant la revendication 6, dans lequel le premier anticorps est associé à une première phase solide en particules et le deuxième anticorps est associé à une deuxième phase solide en particules, et dans lequel la première phase solide en particules est associée à un premier composant d'un système formant un signal et la deuxième phase solide en particules est associée à un deuxième composant du système formant un signal, et dans lequel le premier et le deuxième composants du système formant un signal coopèrent, de manière à créer un signal détectable, si on rapproche l'un de l'autre dans l'espace le premier et le deuxième composants du système formant un signal, et si l'on mesure à l'aide du signal créé l'agglutination des phases solides en particules dans le mélange réactionnel.

10. Procédé suivant la revendication 9, dans lequel le premier composant du système formant un signal est un agent chemioluminescent et le deuxième composant du système formant un signal est un photosensibilisateur ou inversement, et dans lequel on mesure la chemioluminescence dans le mélange réactionnel.

11. Procédé suivant la revendication 1, dans lequel on incube le mélange réactionnel dans le stade b) pendant un laps de temps de 5 secondes à 60 minutes, de préférence de 5 à 20 minutes.

12. Procédé suivant la revendication 1, dans lequel l'activateur de coagulation est un activateur du système de coagulation plasmatique, de préférence du groupe de la thromboplastine, du facteur IIa, du facteur VIIa, du facteur IXa, du facteur Xa, du facteur XIa, du facteur XIIa, des venins de serpent, des phospholipides chargés négativement, des ions calcium, un facteur de tissu, de la silice, du kaolin, de l'acide ellagique, de la célite et du polyphosphate.

13. Procédé suivant la revendication 1, dans lequel l'activateur de coagulation est un activateur à plaquette, de préférence du groupe de l'ADP, de l'épinéphrine, du collagène, du peptide activant un récepteur de la thrombine (TRAP), du U46619 imitant le thromboxane A2, de l'acide arachidonique, de la ristocétine, de la thrombine, du facteur de Willebrand, du peptide se rapportant au collagène (stimulation GPVI), de la convulxine, de l'ionophore de calcium A23187 et du phorbol 12 myristate 13 acétate.

14. Procédé suivant la revendication 1, dans lequel l'activateur de coagulation est un facteur chimique ou physique, qui renforce l'effet procoagulateur d'érythrocyte.

15. Procédé suivant la revendication 1, dans lequel l'activateur de coagulation est un facteur activant les leucocytes.
